# EUROPEAN PATENT SPECIFICATION

(11) **EP 0 686 873 B1**
(45) Date of publication and mention of the grant of the patent: **19.04.2000**
(21) Application number: 95108781.6
(22) Date of filing: 07.06.1995
(51) Int. Cl.: G03C 7/392, G03C 1/005, C07D 303/22, C07D 303/32, C07D 303/34, C07D 303/36, C07D 303/40, C07F 5/02, C07F 9/655

(54) **Color photographic element containing new epoxy scavengers for residual magenta coupler**
Farbphotographisches Element, das neue Epoxy-Abfänger für restlichen Purpurrot-Kuppler enthält
Elément photographique couleur comprenant des agents de blocage époxy nouveaux pour copulant magenta résiduel

(30) Priority: 08.06.1994 US 255512; 25.04.1995 US 427763; 25.04.1995 US 427764; 25.04.1995 US 429269
(43) Date of publication of application: 13.12.1995
(73) Proprietor: EASTMAN KODAK COMPANY, Rochester, New York 14650 (US)
(72) Inventor: Chari, Krishnan, Eastman Kodak Company, Rochester, New York 14650-2201 (US); Smith, Jr., Wendell Franklyn, Eastman Kodak Co., Rochester, New York 14650-2201 (US); Spara, Paul Patrick, Eastman Kodak Company, Rochester, New York 14650-2201 (US); Krishnamurthy, Sundaram, Eastman Kodak Company, Rochester, New York 14650-2201 (US)
(74) Representative: Nunney, Ronald Frederick Adolphe

(56) References cited:
- EP-A- 0 273 412
- WO-A-94/11784
- US-A- 4 540 657
- US-A- 5 300 394

## Description

### Field of the Invention

This invention relates to novel epoxy compounds useful in multilayer silver halide color photographic element, to a color photographic element having improved resistance to thermal and photochemical yellowing, and to a method of preparing said photographic element.

### Background of the Invention

It is well known that thermal and photochemical yellowing are major concerns in magenta image stability of color prints. Over the years improvement in magenta image stability has been achieved by introducing more efficient image stabilizers. However, there still exists a need to further improve the resistance to yellowing in color paper.

It has been known for some time that compounds having the generic structure S are able to undergo reaction with residual magenta coupler and thereby effectively prevent both thermal and photochemical yellowing since the products of the reaction are not yellow and are not prone to yellowing. However, a major problem in the utilization of these compounds is the loss of coupler during storage of the photographic element prior to exposure and processing resulting in a reduction in color density in the print. See for example, U.S. Patent No. 4,540,657 to Krishnamurthy and Japanese Patent Publication No. 62-131259 to Fuji Photo Film Co., Ltd.

Generic structure of *Compound S* is represented below: 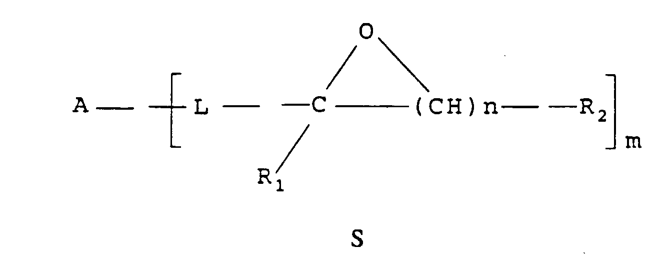 wherein: A is a polyvalent atom, an acidic oxide group, a carboxylic group, a heterocyclic moiety, a carbocyclic group, or an alkane or substituted alkane group; each L is at least one divalent linking group; R₁ and R₂ are H, alkyl, cycloalkyl, aryl, heterocyclic, ester; n is a positive integer with a range of 1-6; m is a positive integer of at least one; with the proviso that at least one of A, L, R₁ or R₂ contains at least one ester or amide group derived from an acidic oxide of carbon, phosphorous, sulfur, boron or silicon.

International Publication WO 94/16363 discloses that the compound S-1 (having the structural formula set forth below) can be incorporated in a silver halide color photographic element containing a ballasted magenta coupler such that there is negligible loss of coupler prior to processing. This was achieved by coating the epoxy compound in a separate layer that was adjacent to the imaging layer containing the magenta coupler and the green sensitized emulsion. Furthermore, it has also been demonstrated that mixing of S-1 with residual magenta coupler after processing may be achieved by using a pH dependent solubilizing agent, e.g., a fatty acid, such as myrisitic acid, in the coating and processing the coating using developer which preferably contains benzyl alcohol. However, the use of benzyl alcohol in the developer raises serious environmental concerns. There is therefore a need to achieve post process mixing of the epoxy scavenger with the residual magenta coupler without using benzyl alcohol in the color developer.

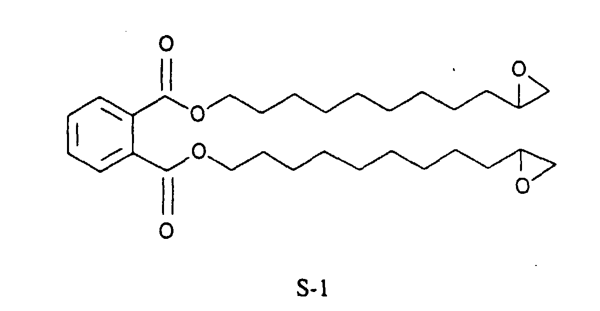

### Problem to be Solved by the Invention

It is desirable to improve the resistance to thermal and photochemical yellowing of color photographic elements, such as color paper, without the use of an external solubilizing agent such as benzyl alcohol.

### Summary of the Invention

An object of this invention is a silver halide based color photographic element having excellent image stability; particularly with respect to thermal and photochemical yellowing.

A further object of this invention is a silver halide based color photographic element containing a scavenger for residual magenta coupler.

A still further object of this invention is a method of incorporating a scavenger for residual magenta coupler in the above photographic element such that loss of coupler prior to processing is minimal.

A still further object of this invention is the use of novel epoxy scavenger compounds having the generic structure SI and a method of incorporating the compounds such that there is elimination of residual magenta after processing the photographic element in a color developer that contains no benzyl alcohol.

These and other objects of the invention are realized by using novel epoxy scavenger compounds that contain a pH dependent ionizable group to facilitate migration of the scavenger from the adjacent scavenger layer (that does not contain any coupler) to the imaging layer (containing coupler and emulsion) at the pH of development.

One aspect of this invention comprises an epoxy compound having the following generic structure SI: 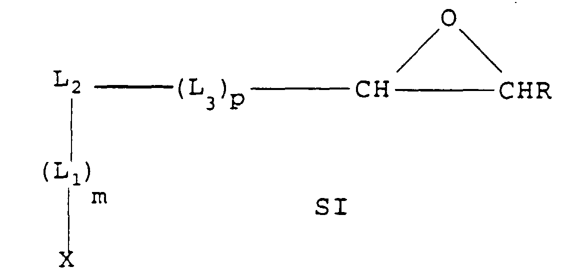 wherein R is H, an alkyl group, or an aryl group; L₁ is an alkyl group or an aryl group; L₂ is -O-, -CO-, -S-, -SO₂-, -PO₂-, -CO₂-, -NHCO- or -NHSO₂-, wherein L₂ may be orientated in either direction; L₃ is an alkyl group; m is 0 or 1; p is 0 or 1; and X is 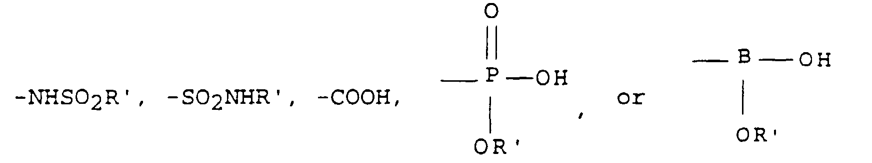 wherein R' is H or an alkyl or aryl group, with the proviso that where L₂ comprises an ionizable group, X may also be an alkyl group or an aryl group.

A further aspect of this invention comprises a color photographic element comprising a support having coated thereon: (a) a photosensitive first layer comprising (i) a silver halide emulsion and (ii) a magenta coupler dispersed therein; and (b) a second layer comprising an epoxy compound of the above formula.

Yet another aspect of this invention comprises a method of preparing a color photographic element comprising coating on a support (a) a photosensitive first layer comprising (i) a silver halide emulsion and (ii) a magenta coupler dispersed therein; and (b) a second layer comprising an epoxy compound of the above formula.

### Advantageous Effect of the Invention

The novel ionizable epoxy compounds can be added to a photographic element to inhibit the thermal and photochemical yellowing of color photographic elements.

### Detailed Description of the Invention

The substituent groups in Formula SI can be unsubstituted or further substituted with photographically acceptable substituents. Typical examples of photographic substituents include alkyl, aryl, anilino, carbonamido, sulfonamido, alkylthio, arylthio, alkenyl, cycloalkyl, and further to these exemplified are halogen, cycloalkenyl, alkinyl, heterocyclyl, sulfonyl, sulfinyl, phosphonyl, acyl, carbamoyl, sulfamoyl, cyano, alkoxy, aryloxy, heterocyclyloxy, siloxy, acyloxy, carbamoyloxy, amino, alkylamino, imido, ureido, sulfamoylamino, alkoxycarbonylamino, aryloxycarbonylamino, alkoxycarbonyl, aryloxycarbonyl, heterocyclylthio, spiro compound residues and bridged hydrocarbon compound residues. Usually the substituent will have less than 30 carbon atoms and typically less than 20 carbon atoms. It is understood throughout this specification that any reference to a substituent by the identification of a group containing a substitutable hydrogen (e.g. alkyl, amine, aryl, alkoxy, heterocyclic), unless otherwise specifically stated, shall encompass not only the substituent's unsubstituted form, but also its form substituted with any other photographically useful substituents. Preferred substituents on the alkyl and aryl groups of Formula SI are hydrocarbyl groups, one or more hetero atoms, such as chlorine or one or more hetero groups containing for example, N, P, S.

R in Formula SI is preferably H or alkyl, such as methyl, ethyl. Preferred compounds of Formula SI for use in the invention include those wherein m and p are each 1, L₁ is phenyl, L₂ is -O-, -CO-, -SO₂-, -PO₂-, or -CO₂-, L₃ is a linear or branched alkyl group, X is -NHSO₂R', -SO₂NHR', -COOH, 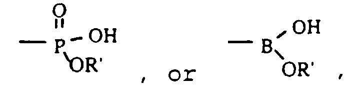 and R' is an alkyl or aryl group.

One class of preferred compounds of Formula SI for use in the invention include those compounds of the formula SI-A: 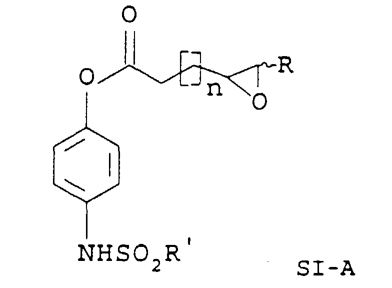 wherein: R is H or an alkyl or aryl group; R' is H or an alkyl or aryl group; and n is 2 to 20. R is preferably H or alkyl, such as methyl. R' is preferably substituted phenyl, such as p-chlorophenyl.

A second class of preferred compounds are those of formula SI wherein X is 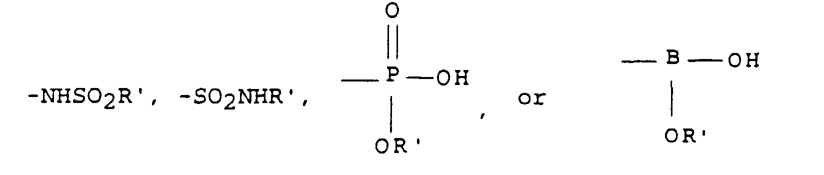 wherein R' is H or an alkyl or aryl group, with the proviso that where L₂ comprises an ionizable group, X may also be an alkyl group or an aryl group; and wherein the compound has a melting point of less than about 50°C. Particularly preferred are those having the generic structural formula SI-B: 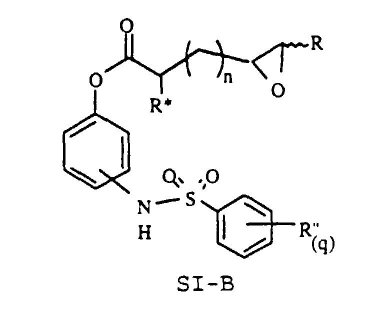 wherein: R* is H or an alkyl or aryl group; n is from 1 to about 20; q is 1, 2, or 3; and each R'' is H, an alkoxide group, a phosphate group, a sulfate group, a sulfonamide group, a sulfone group, a halogen atom, or an alkyl group; with the proviso the appended sulfonamido group is in a meta or para position with respect to the -CO₂- linking group, and with the further proviso when the appended sulfonamido group is in the para position each R'' is H, or at least one appended R'' group which is not H is in the meta or ortho position with respect to the -NHSO₂- linking group, or R* is not H.

Substitution in the ortho or meta position on the phenyl rings in formula SI-B has been found to generally result in lower melting points than comparable para-substituted compounds, and the addition of a chiral center (a carbon atom having four different substituents, such as where R* is other than H) also tends to lower melting point in comparison to analogous compounds. Additionally, compounds wherein each R'' is H (i.e., wherein the phenyl substituent is unsubstituted) have also been found to result in generally lower melting points than compounds wherein the phenyl ring is substituted. Accordingly, compounds satisfying the above preferred provisos have been found to generally have lower melting points than compounds which do not meet such provisos.

A general scheme for the synthesis of preferred compounds of formula SI is given below:

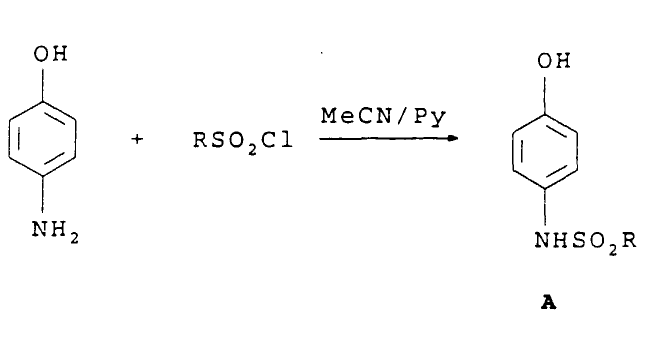

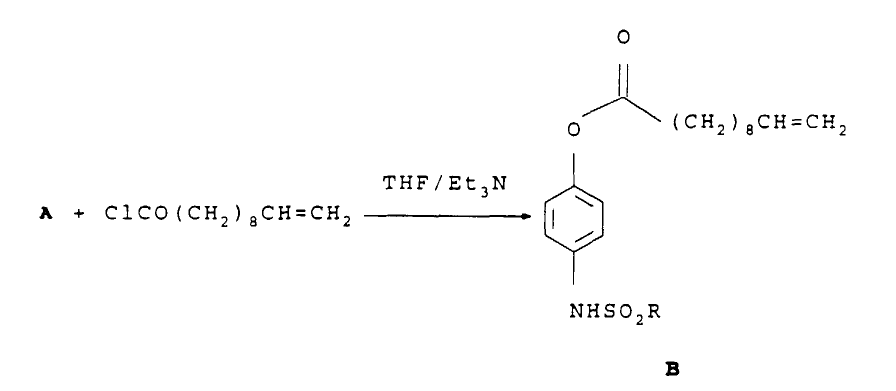

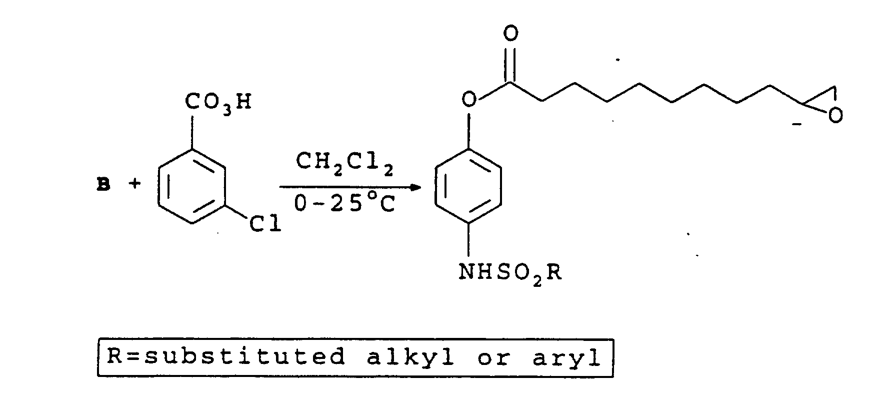

Illustrative epoxy compounds of this invention are:

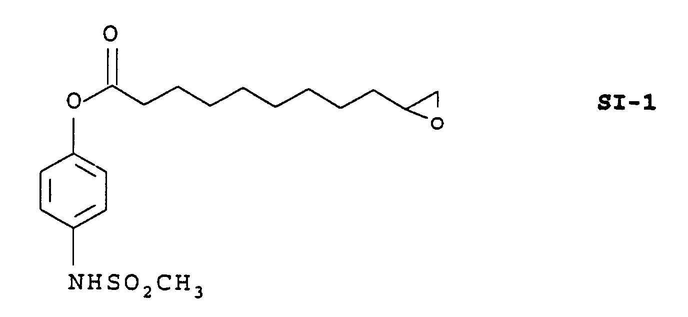

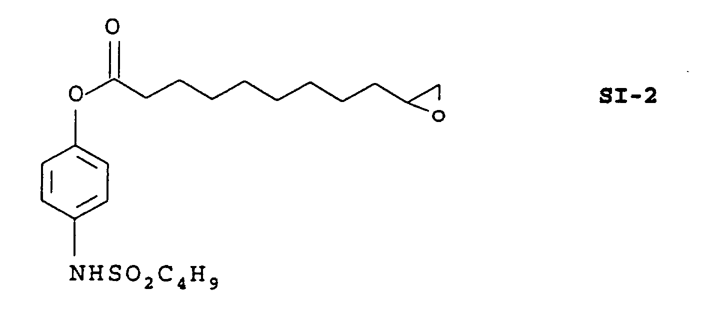

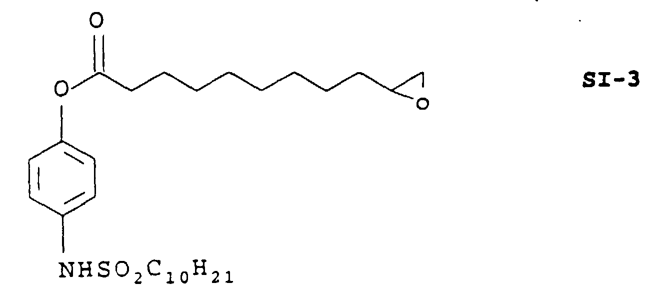

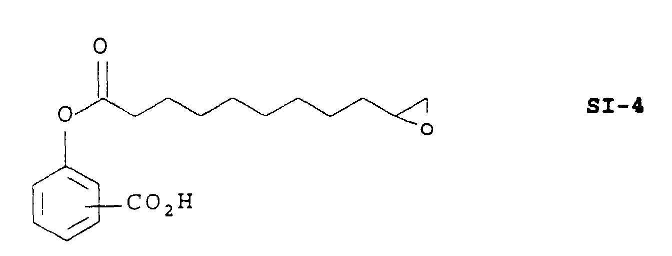

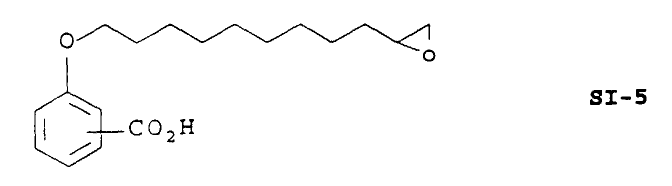

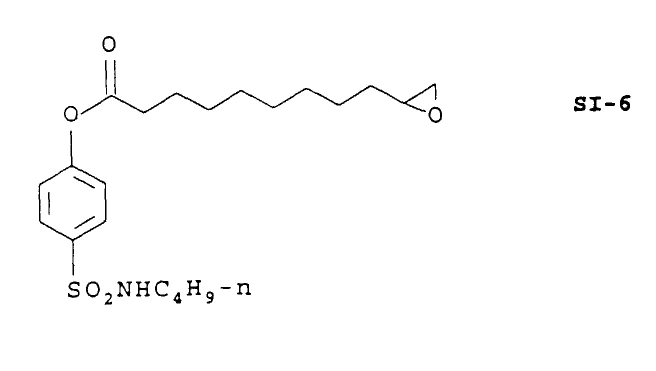

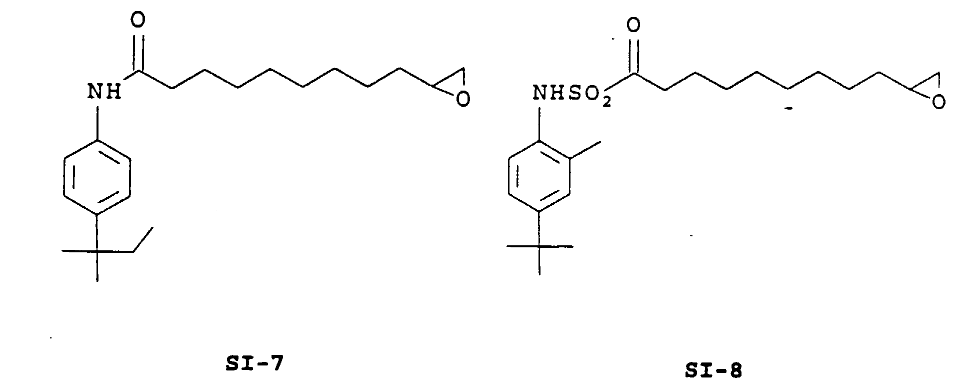

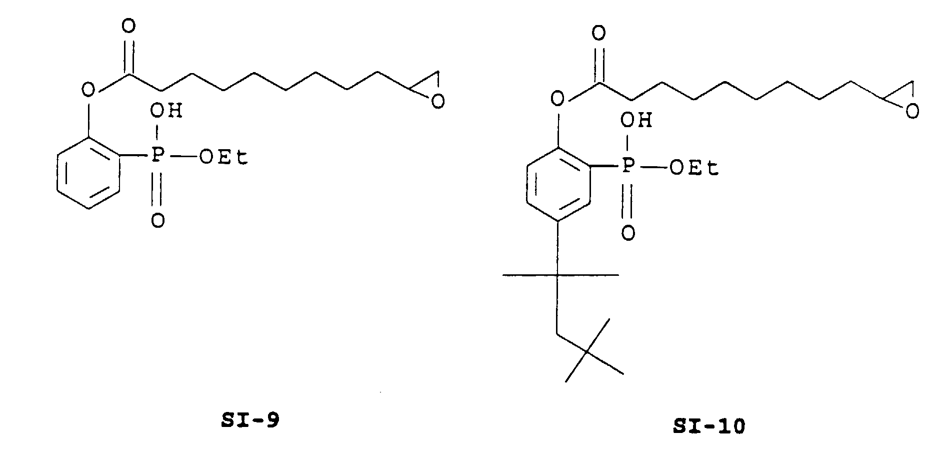

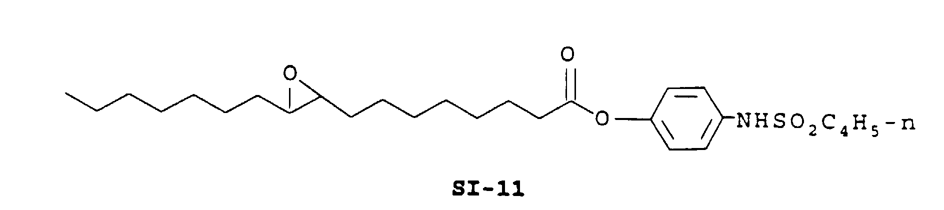

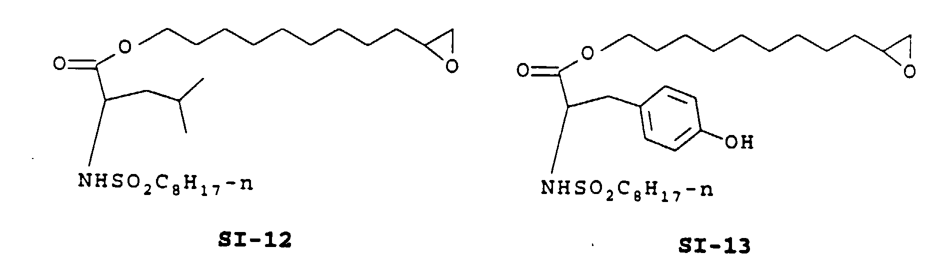

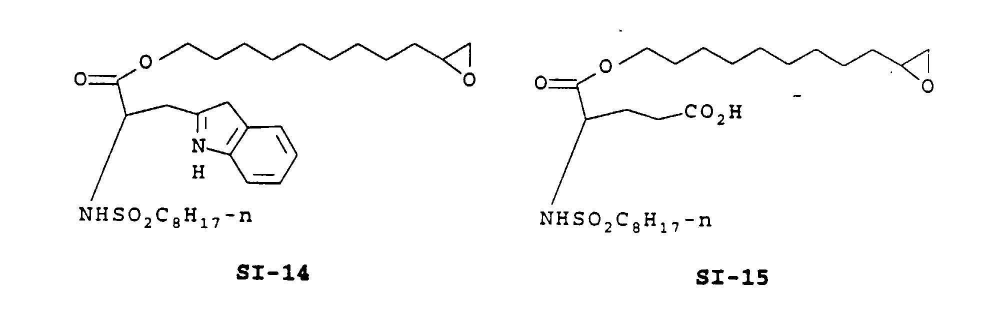

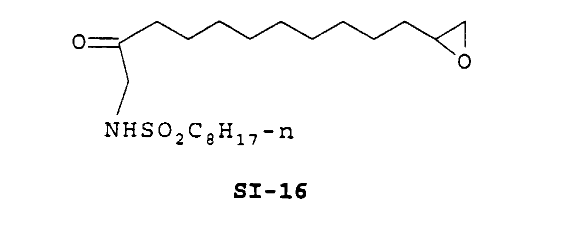

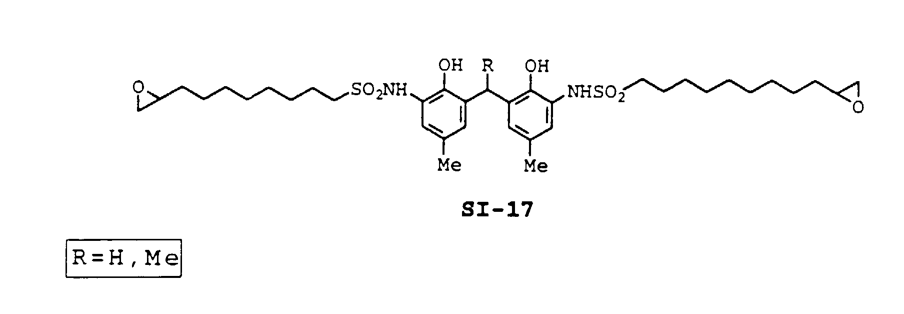

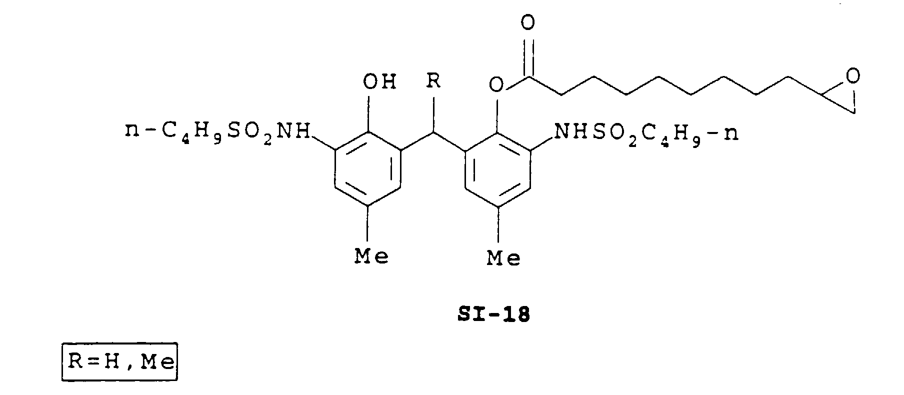

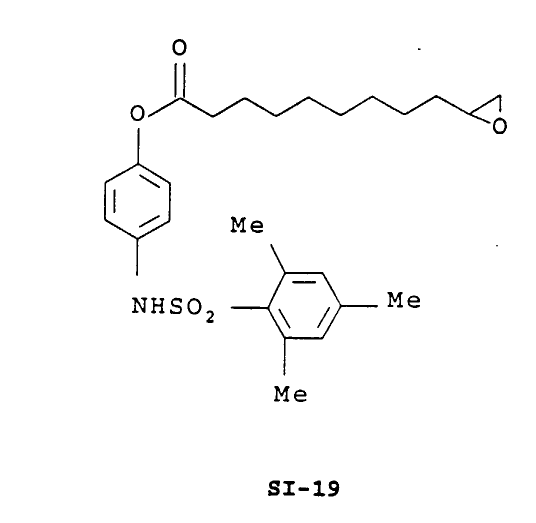

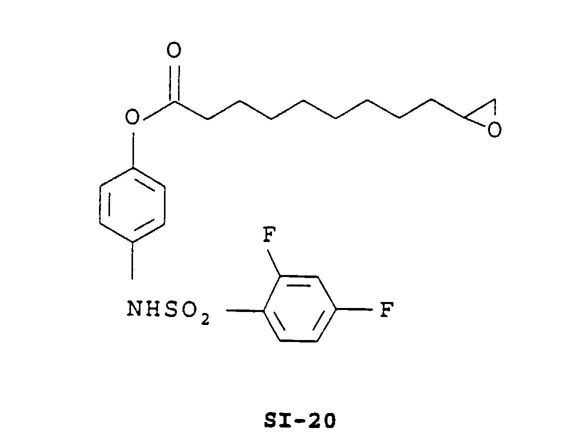

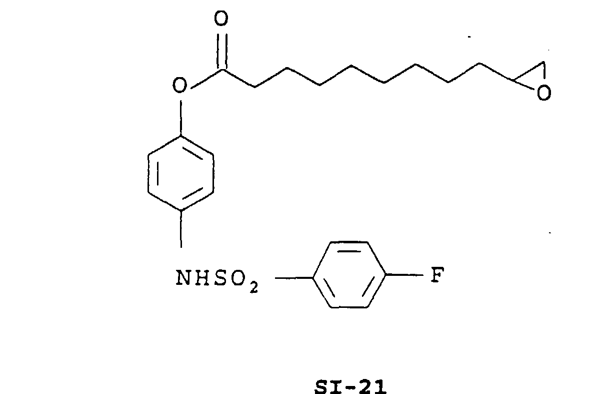

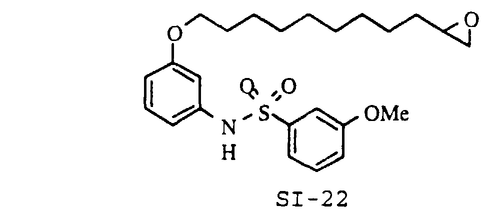

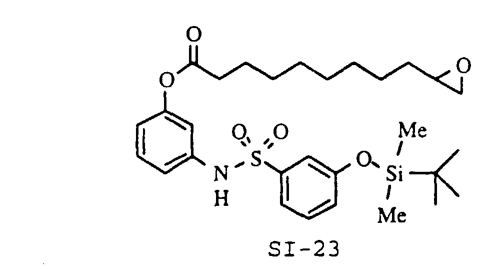

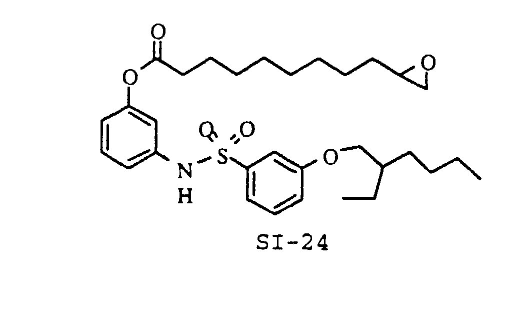

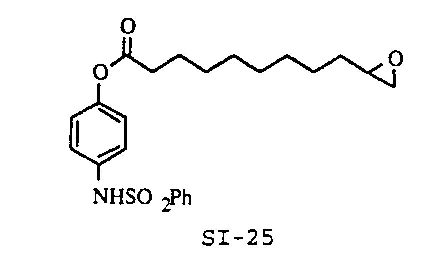

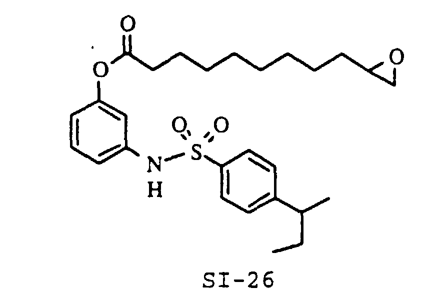

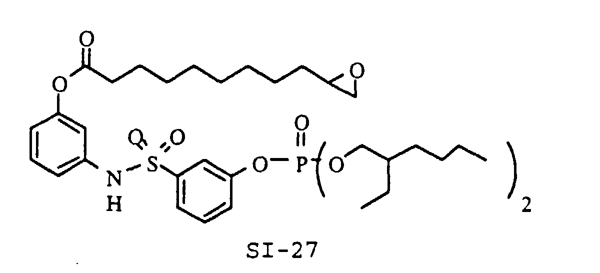

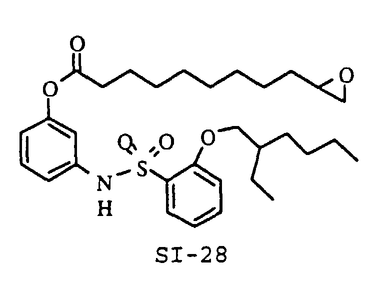

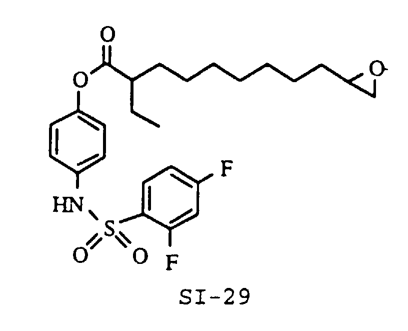

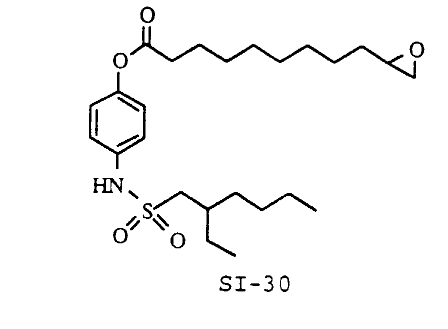

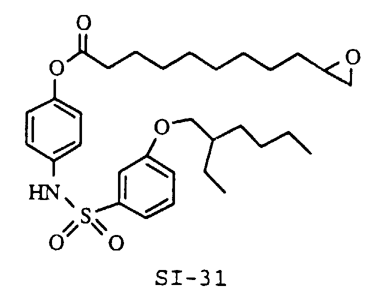

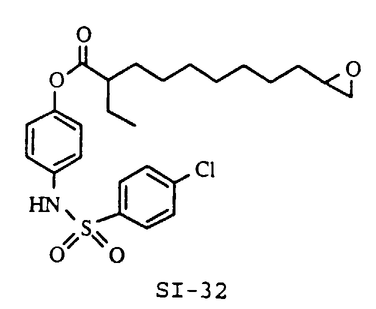

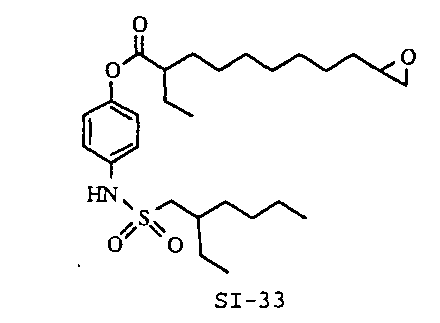

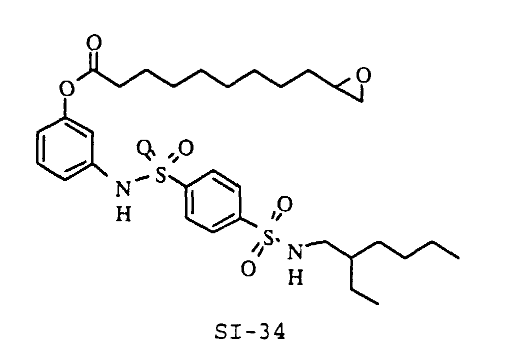

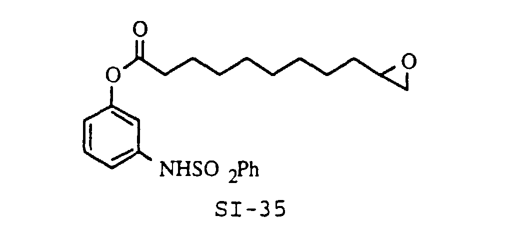

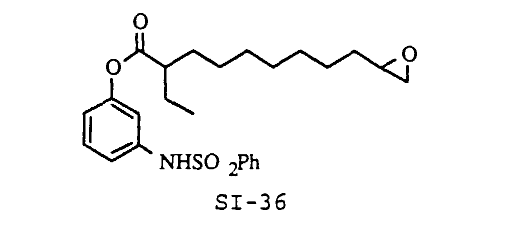

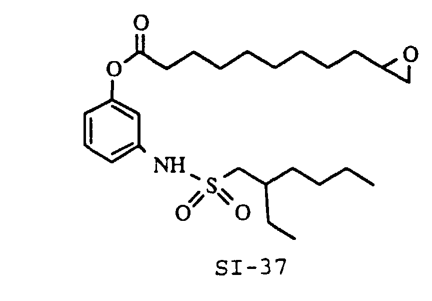

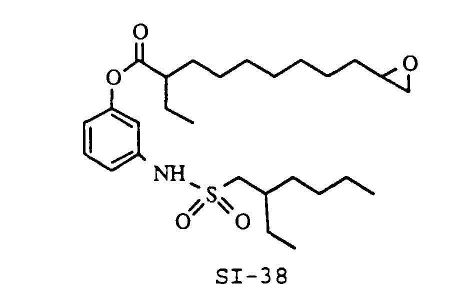

Specific compounds of a preferred structure of SI are formula SI-C : 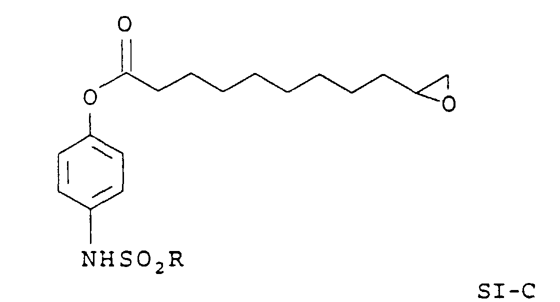 wherein R is an alkyl or phenyl group. Examples of such compounds are as follows:

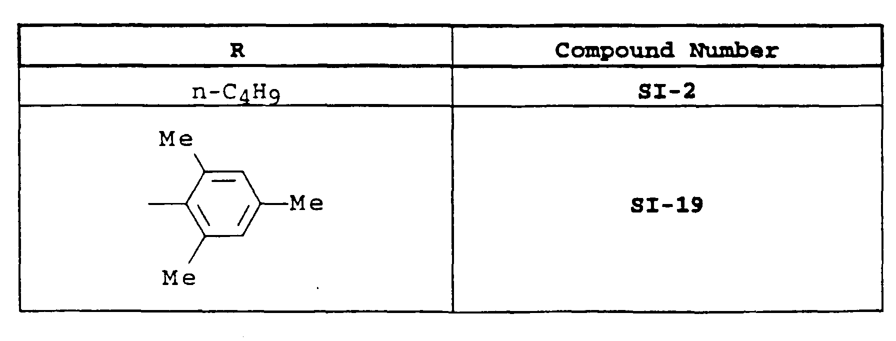

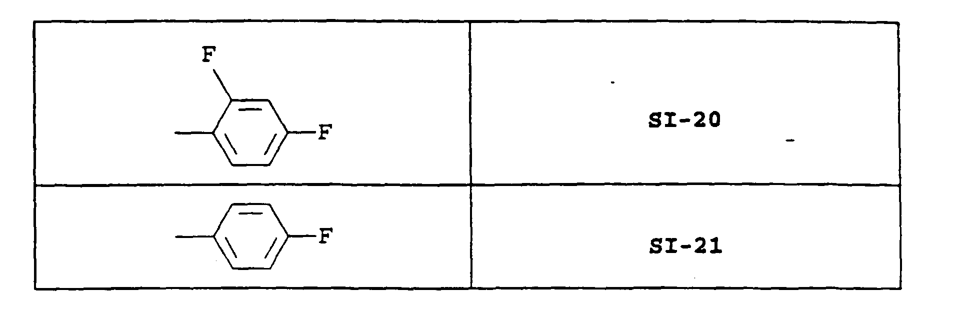

Physical properties of selected SI compounds having the formula SI-C with R as defined in the following table are shown below.

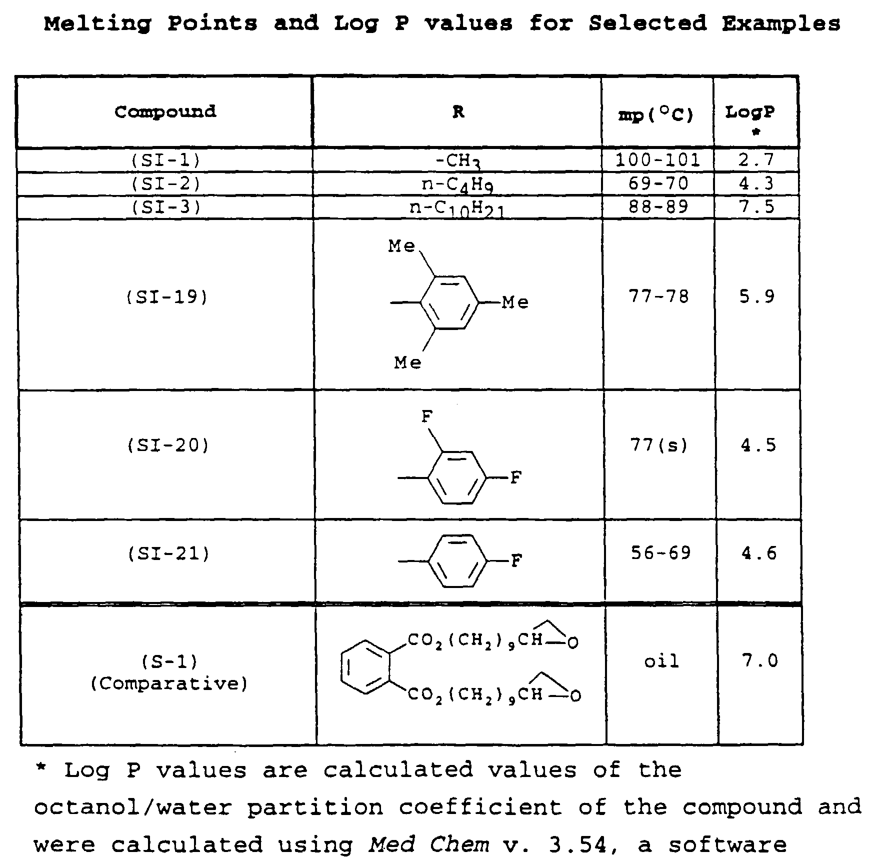

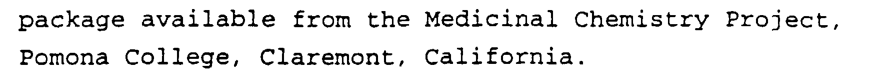

Dispersions of compounds of Formula SI may be in the form of solid particle dispersions (e.g., dispersions of minute solid particles formed by ball-milling such compounds), or oil-in-water dispersions. Such dispersions are formed by dispersing either solid particles of compounds of Formula SI or an organic liquid phase of the compound (optionally with an organic solvent) in an aqueous solution.

Where it is desired to incorporate the compounds of the invention into photographic elements in the form of solid particles, solid particle dispersions may be made by mixing the solid of interest with an aqueous solution that may contain one or more grinding aids or stabilizers. Particle size reduction is accomplished by subjecting the solid crystals in the slurry to repeated collisions with hard, inorganic milling media, such as sand, spheres of silica, stainless steel, silicon carbide, glass, zirconium, zirconium oxide, yttria-stabilized zirconium oxide, alumina, titanium etc., all of which fracture the crystals. The bead sizes typically range from 0.25 to 3.0 millimeters (mm) in diameter. Ball mills, media mills, attritor mills, jet mills, vibratory mills, etc. are frequently used to accomplish particle size reduction.

It has been found that there may be a loss in color density in the high exposure (or highlight) regions of a color print if materials comprising solid particle dispersions of compounds of formula SI are stored at elevated temperatures for long periods of time prior to exposure and processing. The latter is believed to result from unwanted migration of the epoxy compound from the adjacent nonimaging layer to the magenta imaging layer prior to processing. We have found that certain components can effectively improve raw stock keeping of photographic elements comprising solid particle dispersions of residual magenta coupler scavenger epoxy compounds. The components are hydrophobic, photographically inert compounds that have a logP_{(calc)} of at least about 6, more preferably at least about 8 and most preferably at least about 9, where logP_{(calc)} is the logarithm of the value of the octanol/water partition coefficient (P) of the compound calculated using MedChem, version 3.54, a software package available from the Medicinal Chemistry Project, Pomona College, Claremont, California. Such compounds preferably have a solubility in water of less than 1.0 µg/mL. LogP_{(calc)} is a parameter which is highly correlated with measured water solubility for compounds spanning a wide range of hydrophobicity. Compounds having a high logP_{(calc)} may be so highly hydrophobic that it is difficult to measure their water solubility using standard techniques. LogP_{(calc)} is a useful means to characterize their hydrophobicity.

In accordance with a preferred embodiment of this invention, raw stock keeping of photographic elements incorporating milled solid particles of epoxy compounds of formula SI can be improved by the use of hydrophobic, photographically inert liquid substances during the milling process. The hydrophobic, photographically inert compounds used in this invention are liquids during milling and have a logP_{(calc)} greater than about 6. Preferred hydrophobic, photographically inert compounds are those selected from the following classes of compounds:
I. alkanes, alkenes or alkyl halides having a logP_{(calc)} greater than about 6,
II. compounds which have an elemental composition consisting of carbon, hydrogen, and oxygen and a logP_{(calc)} greater than about 6,
III. Esters and amides of sulfur or phosphorous acids having a logP_{(calc)} greater than about 6,
IV. Amides and amines having a logP_{(calc)} greater than about 6.

Representative compounds are given below, along with their logP_{(calc)} value. Each Log P_{(calc)} was calculated using the above-mentioned MedChem software package (version 3.54). This software package is well known and accepted in the chemical and pharmaceutical industries.

Compounds of class I include: straight or branched chain alkanes and alkenes such as, for example, hexadecane and octadecene, and haloalkanes such as hexadecyl bromide and octadecyl chloride.

Compounds of class II include any liquid with a calculated logP_{(calc)} greater than about 6 and with an elemental composition consisting of carbon, hydrogen, and oxygen. Such compounds include, for example, diesters such as bis(2-ethylhexyl) azelate, substituted aromatic compounds such as phthalates, isophthalates, and terephthalates, including for example, dinonyl phthalate, didecylphthalate, and didodecylphthalate.

Compounds of class III include esters and amides of sulfur or phosphorous acids including, for example, sulfates, sulfonates, sulfonamides, phosphates, phosphonates, phosphites, and phosphine oxides. Particular examples include triesters of phosphoric acid, such as tri(2-ethylhexyl) phosphate, and trisubstituted phosphine oxides, such as trioctylphosphine oxide.

Compounds of class IV include, for example, trioctyl amine.

Representative compounds and their respective logP_{(calc)} values are given below:
1. hexadecane (9.16)
2. bis (2-ethylhexyl)azelate (9.20)
3. tri (2-ethylhexyl) phosphate (9.49)
4. trioctylphosphine oxide (9.74)
5. didecyl phthalate (11.04)
6. trioctyl amine (10.76)
7. tritolyl phosphate (6.58)
8. 2,4-di-tert-pentylphenol (6.49)
9. 1,4-cyclohexanedimethanol bis(2-ethylhexanoate) (8.14)
10. oleyl alcohol (7.69)
11. p-dodecylphenol (7.94)
12. trihexyl phosphate (6.70)
13. isopropyl palmitate (8.39)
14. dihexyl hexylphosphonate (6.32)
15. dodecylbenzene (8.61)

Some of the hydrophobic, photographically inert components useful in the practice of this invention have been disclosed to be useful in photographic dispersions as permanent solvents for couplers or other photographically useful compounds. Such use is directed towards conventional oil-in-water dispersions, rather than the solid particle dispersions of the invention.

The dispersions of this embodiment of the invention are formed by milling an epoxy compound of formula SI in the presence of a photographically inert liquid compound. Where the melting point of the photographically inert compound is below room temperature (which is preferred), milling may be done at room temperature. Alternatively, milling may be performed at elevated temperatures above the melting point of photographically inert compounds which are not liquids at room temperature. While the hydrophobic, photographically inert substance in accordance with the invention is used at a sufficient level to have an effect on the raw stock keeping (generally above 0.01 wt% based upon the weight of the milled epoxy compound, preferably above 1 wt%), the preferred amount of hydrophobic, photographically inert substance for use in this invention is a level less than the total amount of epoxy compound of formula SI. The more preferred level of hydrophobic, photographically inert substance is less than one half the weight of the epoxy compound of formula SI. The most preferred level of hydrophobic, photographically inert compound is less than or about one fourth the weight of the epoxy compound of formula SI.

A preferred process in accordance with one embodiment of the invention comprises adding an organic compound of Formula SI in a liquid state to an aqueous solution, and directly dispersing the compound in the aqueous solution. The dispersions may be prepared by means known in the art, such as by mixing under conditions of high shear or turbulence. Where it is desired to form oil-in water dispersions, while relatively high-melting (i.e., having a melting point above about 50°C) compounds of formula SI may be employed with the use of sufficient solvent amounts, compounds of Formula SI which are liquid at room temperature, or which are relatively low-melting (i.e., having a melting point below about 50°C), are preferably employed in order to obtain stable dispersions. Compounds of formula SI-B are particularly suitable for formation of oil-in-water dispersions.

While the low melting compounds of formula SI-B are preferably liquid at room temperature, or may be liquefied at moderately raised temperatures, the liquid state organic, or oil phase, components of such dispersions may additionally include high-boiling organic solvents, known as oil formers, coupler solvents, or permanent solvents. High boiling solvents have a boiling point sufficiently high, generally above 150°C at atmospheric pressure, such that they are not evaporated under normal dispersion making and photographic layer coating procedures. It is an advantage of one embodiment of the invention that the low melting compounds form dispersions which are more stable that comparative high-melting compound dispersions formed with organic solvents.

Non-limitive examples of high boiling organic solvents that may be used include the following: Dibutyl phthalate, Tritolyl phosphate, N,N-Diethyldodecanamide, Tris(2-ethylhexyl)phosphate, Octyl oleate monoepoxide, 2,5-Di-t-pentylphenol, Acetyl tributyl citrate, 1,4-Cyclohexylenedimethylene bis(2-ethylhexanoate), Bis(2-ethylhexyl) phthalate, 2-phenylethyl benzoate, Dibutyl sebacate, N,N-Dibutyldodecanamide, Oleyl alcohol, 2-(2-Butoxyethoxy)ethyl acetate.

Auxiliary solvents may also be included in dispersion making processes. Many useful auxiliary solvents are water immiscible, volatile solvents, and solvents with limited water solubility which are not completely water miscible. Examples of these include the following: Ethyl acetate, Cyclohexanone, 4-Methyl-2-pentanol, Triethyl phosphate, Methylene chloride, Tetrahydrofuran.

The aqueous phase of the dispersions used in the invention may comprise a hydrophilic colloid, preferably gelatin. This may be gelatin or a modified gelatin such as acetylated gelatin, phthalated gelatin, oxidized gelatin, etc. Gelatin may be base-processed, such as lime-processed gelatin, or may be acid-processed, such as acid processed ossein gelatin. The hydrophilic colloid may be another water-soluble polymer or copolymer including, but not limited to poly(vinyl alcohol), partially hydrolyzed poly(vinylacetate/vinylalcohol), hydroxyethyl cellulose, poly(acrylic acid), poly(1-vinylpyrrolidone), poly(sodium styrene sulfonate), poly(2-acrylamido-2-methane sulfonic acid), polyacrylamide. Copolymers of these polymers with hydrophobic monomers may also be used.

The dispersions and coated layers of the elements of the invention may include surfactants. Surfactants may be cationic, anionic, zwitterionic or non-ionic. Ratios of surfactant to liquid organic solution typically are in the range of 0.5 to 25 wt.% for forming small particle photographic dispersions, which ratios are also useful for forming the invention dispersions. Useful surfactants include, but are not limited the following.

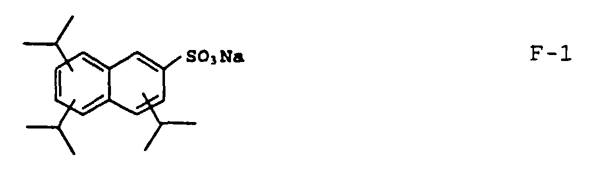

**CF**_{**3**}**(CF**_{**2**}**)**_{**7**}**SO**_{**3**}**Na** F-2

**CH**_{**3**}**(CH**_{**2**}**)**_{**n**}**SO**_{**3**}**Na, n = 12-14** F-3

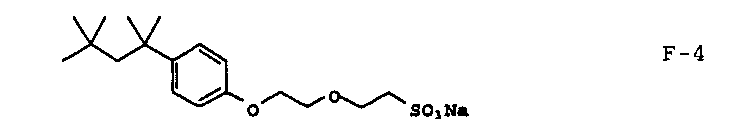

**CH**_{**3**}**(CH**_{**2**}**)**_{**11**}**OSO**_{**3**}**Na** F-5

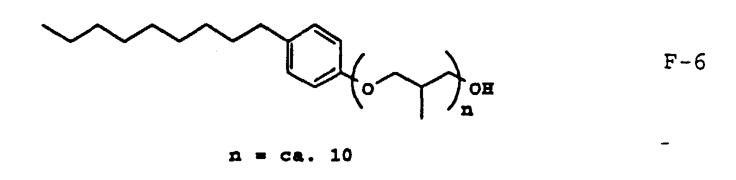

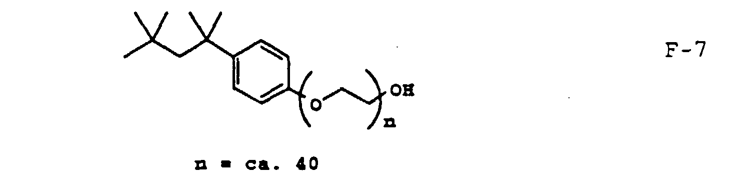

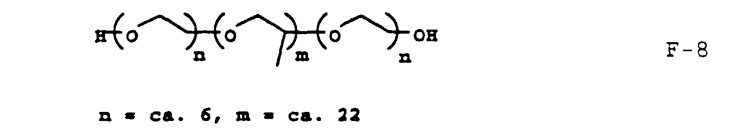

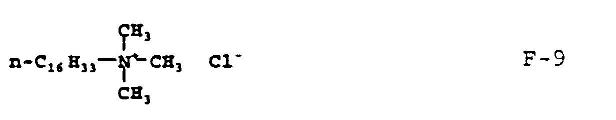

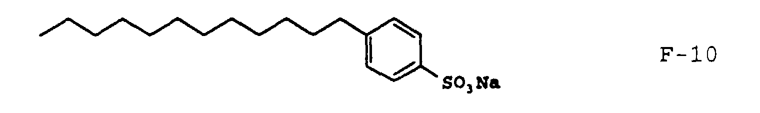

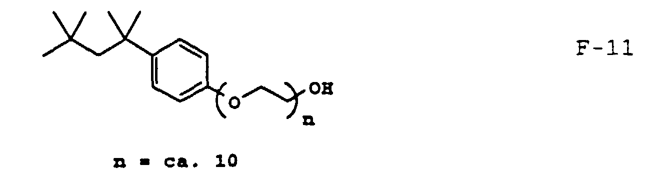

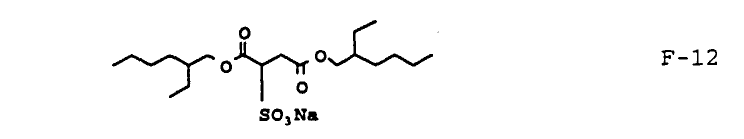

Devices suitable for the high-shear or turbulent mixing of the dispersions of the invention include those generally suitable for preparing submicron photographic emulsified dispersions. These include but are not limited to blade mixers, devices in which a liquid stream is pumped at high pressure through an orifice or interaction chamber, sonication, Gaulin mills, homogenizers, blenders, etc. More than one type of device may be used to prepare the dispersions.

It has been found that many conventional compounds which are used as image light stabilizers for magenta image dyes can severely inhibit the post-process reaction between residual magenta coupler and the epoxy compounds of the invention and thereby suppress the beneficial effects of the epoxy compounds on yellowing. In a preferred embodiment of the invention, the epoxy compounds are used in combination with image stabilizers for the magenta image dye such that there is little or no inhibition of the post-process reaction between the epoxy compound and residual magenta coupler. Such embodiment comprises using an image stabilizer of the following formula STG-A in the photosensitive layer of the elements of the invention comprising a magenta coupler dispersion. 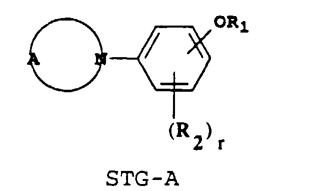 wherein R₁ represents an alkyl group, a cycloalkyl group, an alkenyl phenyl group, an aryl group, a heterocyclic group, an acyl group, a bridged hydrocarbon group, an alkyl sulfonyl group or an aryl sulfonyl group; R₂ represents a group capable of being substituted on the benzene ring; r represents an integer between 0 and 4; and A represents a group of non metal atoms necessary for the formation of a 5 to 8 membered ring together with the nitrogen atom.

A in formula STG-A preferably contains an electron withdrawing group. Most preferably A contains either a sulfone or sulfoxide group. For R₁, the alkyl group may include, e.g., a straight-chain or branched-chain alkyl group having 1 to 24 carbon atoms; the cycloalkyl group, e.g., a cycloalkyl group having 5 to 24 carbon atoms; the alkenyl group, e.g., an alkenyl group having 3 to 24 carbon atoms; the aryl group, e.g., a phenyl group and naphthyl group; the heterocyclic group, e.g., a pyridyl group, an imidazolyl group and a thiazole group; the acyl group, e.g., an acetyl group and a benzoyl group; the bridged hydrocarbon group, e.g., a bicyclo[2.2.1]heptyl group, etc., respectively. R₂ represents, e.g., a halogen atom and the groups such as alkyl, aryl, alkoxy, aryloxy, alkythio, arylthio, acyl, alkoxycarbonyl, carbamoyl (e.g., alkylcarbamoyl, arylcarbamoyl) ureido (e.g., alkylureido, arylureido), sulfamoyl (e.g., alkylsufamoyl, arylsulfamoyl), amino, alkylsulfonyl, arylsulfonyl, nitro, cyano and carboxy.

Preferred compounds of Formula STG-A are illustrated below.

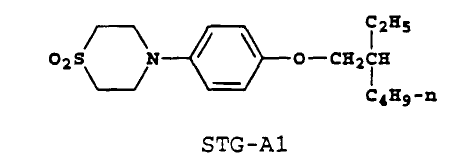

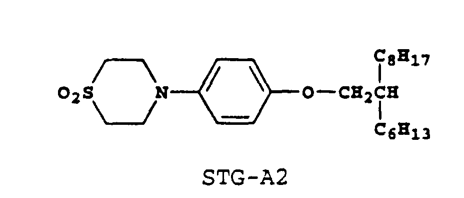

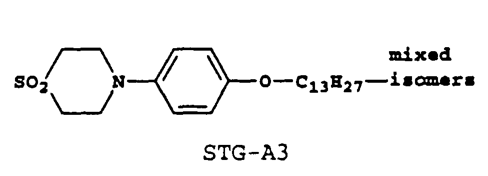

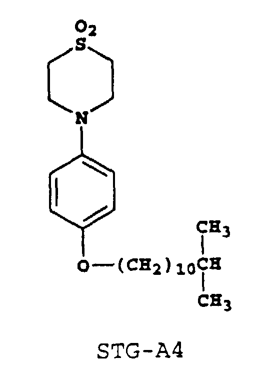

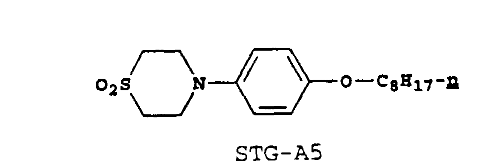

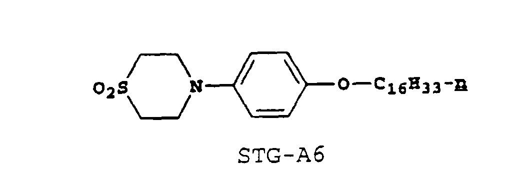

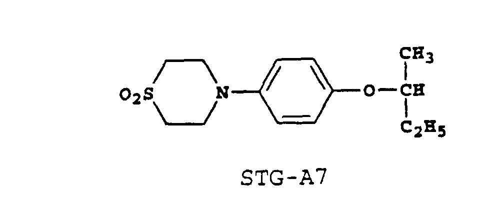

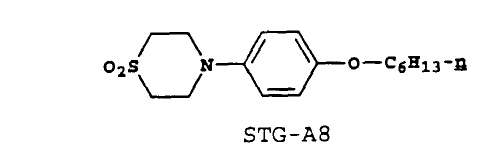

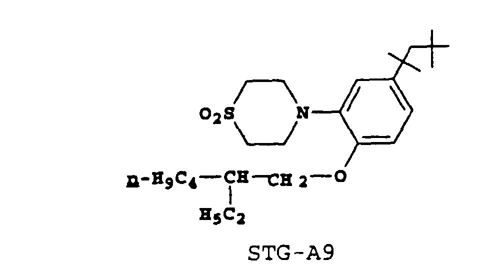

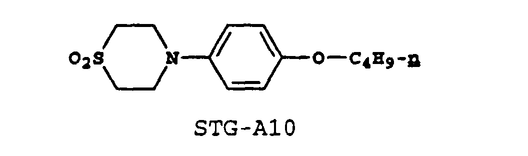

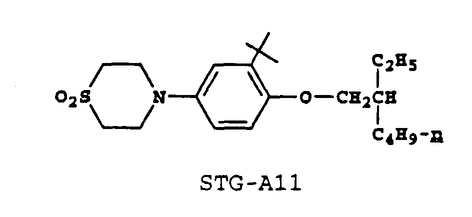

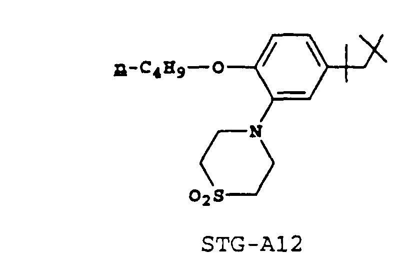

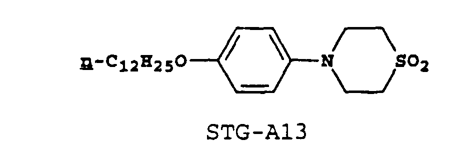

The image stabilizers of Formula STG-A may be synthesised by by well-known techniques, such as those described in U.S. Pat. No. 5,017,465.

The magenta dye forming coupler is preferably a pyrazolone, pyrazolotriazole, pyrazolobenzimidazole with or without a suitable leaving group. The magenta coupler can be monomeric, dimeric, trimeric, oligomeric or polymeric coupler wherein the coupler moiety can be attached to the polymeric backbone via a substituent on the coupler moiety or a substituent on a coupling off group. Illustrative magenta couplers are disclosed in, for example, U.S. Patents Nos. 1,969,479; 2,311,082; 2,343,703; 2,369,489; 2,575,182; 2,600,788; 2,706,685; 2,908,573; 3,061,432; 3,062,653; 3,152,896; 3,153,816; 3,214,437; 3,253,924; 3,311,476; 3,419,391; 3,519,429; 3,725,067; 3,770,447; 3,907,571; 3,928,044; 3,935,015; 4,120,723; 4,123,281; 4,199,361; 4,336,325; 4,351,897; 4,385,111; 4,401,752; 4,407,936; 4,413,054; 4,283,472; 4,338,393; 4,420,556; 4,443,536; 4,500,630; 4,522,915; 4,540,654; 4,576,912; 4,581,326; 4,621,046; 4,728,598; 4,774,172; and 4,853,319 European Patent Applications Nos. 284,239; 284,240; 240,852; 170,164; and 177,765; Japanese Patent Publication Nos. 60/170854, 60/194451 and 60/194452 and Great Britain Patents Nos. 1,047,612, 1,357,372 and 1,530,272, and "Farbkuppler-eine Literaturübersicht", published in Agfa Mitteilungen, Band III, pp 126-156 (1961); the disclosures of which are incorporated herein by reference.

Magenta dye-forming couplers comprise pyrazolone compounds of the general formulae: 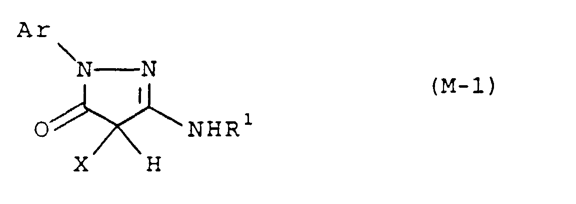 and 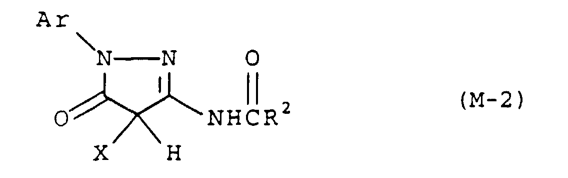 pyrazolotriazole compounds of the general formulae: 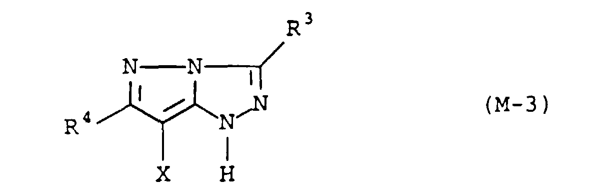 and 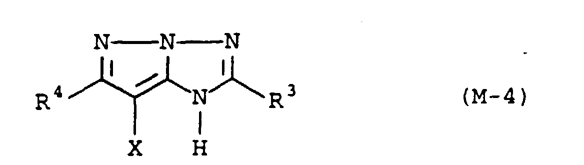 and pyrazolobenzimidazoles of the formula: 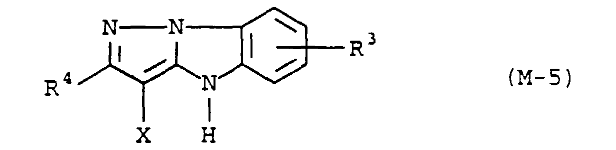 wherein
Ar is an unsubstituted aryl group or an aryl group (including pyridyl) substituted with one or more substituents selected from halogen atoms and cyano, alkylsulfonyl, arylsulfonyl, sulfamoyl, sulfonamido, carbamoyl, carbonamido, alkoxy, acyloxy, aryloxy, alkoxycarbonyl, aryloxycarbonyl, ureido, nitro, alkyl, and trifluoromethyl, or Ar is an aryl group substituted with a group which forms a link to a polymeric chain;
R¹ is a substituted or unsubstituted phenyl group and R² is a substituted or unsubstituted alkyl or phenyl group, the R¹ and R² substituents being individually selected from halogen atoms, and alkyl, aryl, alkoxy, aryloxy, carbonamido, carbamoyl, sulfonamido, sulfamoyl, alkylsulfinyl, arylsulfinyl, alkylsulfonyl, arylsulfonyl, alkoxycarbonyl, aryloxycarbonyl, acyl, acyloxy, ureido, imido, carbamate, heterocyclic, cyano, trifluoromethyl, alkylthio, nitro, carboxyl and hydroxyl groups, provided that R¹ and R² each contain at least 6 carbon atoms or the R¹ and R² substituents may individually comprise a group which forms a link to a polymeric chain;
R³ and R⁴ are individually selected from the group consisting of hydrogen, substituted and unsubstituted alkyl, substituted and unsubstituted phenyl, substituted and unsubstituted alkoxy, substituted and unsubstituted amino, substituted and unsubstituted anilino, substituted and unsubstituted acylamino, halogens and a group which links to a polymer, provided that the total number of carbon atoms contained in R³ and R⁴ is at least 6 if neither R³ nor R⁴ is a group which links to a polymer; and
X is hydrogen or a coupling-off group selected from the group consisting of halogens, alkoxy, aryloxy, alkylthio, arylthio, acyloxy, sulfonamido, carbonamido, arylazo, nitrogen-containing heterocyclic and imido groups. Coupling-off groups are well known to those skilled in the photographic art. Generally, such groups determine the equivalency of the coupler and modify the reactivity of the coupler. Coupling-off groups can also advantageously effect the layer in which the coupler is coated or other layers in the photographic material by performing, after release from the coupler, such functions as development inhibition, bleach acceleration, color correction, development acceleration and the like. Representative coupling-off groups include, as noted above, halogens (for example, chloro), alkoxy, aryloxy, alkyl thio, aryl thio, acyloxy, sulfonamido, carbonamido, arylazo, nitrogen-containing heterocyclic groups such as pyrazolyl and imidazolyl, and imido groups such as succinimido and hydantoinyl groups. Except for the halogens, these groups may be substituted if desired. Coupling-off groups are described in further detail in: U.S. Patents Nos. 2,355,169; 3,227,551; 3,432,521; 3,476,563; 3,617,291; 3,880,661; 4,052,212 and 4,134,766, and in British Patent References Nos. 1,466,728; 1,531,927; 1,533,039; 2,006,755A and 2,017,704A, the disclosures of which are incorporated herein by reference.

Preferred structures of magenta couplers are 4- or 2-equivalent pyrazolone couplers, particularly couplers of the structure: 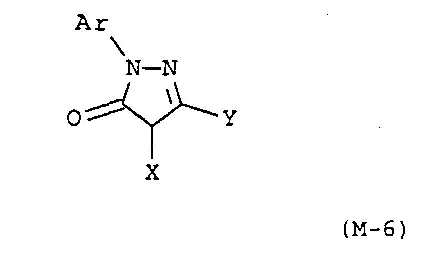 wherein:
Ar is selected from the group consisting of unsubstituted aryl groups, substituted aryl groups and substituted pyridyl groups, the substituents being selected from the group consisting of halogen atoms and cyano, alkylsulfonyl, arylsulfonyl, sulfamoyl, sulfamido, carbamoyl, carbonamido, alkoxy, acyloxy, aryloxy, alkoxycarbonyl, aryloxycarbonyl, ureido, nitro, alkyl and trifluoromethyl groups;
Y is an anilino group substituted with one or more substituents selected from the group consisting of halogen atoms, and alkyl, aryl, alkoxy, aryloxy, carbonamido, carbamoyl, sulfonamido, sulfamoyl, alkylsulfinyl, arylsulfinyl, alkylsulfonyl, arylsulfonyl, alkoxycarbonyl, aryloxycarbonyl, acyl, acyloxy, ureido, imido, carbamate, heterocyclic, cyano, hydroxyl groups, and groups which form a link to a polymeric chain, and wherein Y contains at least 6 carbon atoms; and
X is a coupling-off group selected from the group consisting of halogen, alkoxy, aryloxy, alkylthio, arylthio, acyloxy, sulfonamido, sulfonyloxy, carbonamido, arylazo, nitrogen-containing heterocyclic and imido groups.
Coupling-off groups are well known to those skilled in the photographic art. Generally, such groups determine the equivalency of the coupler and modify the reactivity of the coupler. Coupling-off groups can also advantageously effect the layer in which the coupler is coated or other layers in the photographic material by performing, after release from the coupler, such functions as development inhibition, bleach acceleration, color correction, development acceleration and the like. Representative coupling-off groups include, as noted above, halogens (for example, chloro), alkoxy, aryloxy, alkylthio, arylthio, acyloxy, sulfonamido, carbonamido, arylazo, nitrogen-containing heterocyclic groups such as pyrazolyl and imidazolyl, and imido groups such as succinimido and hydantoinyl groups. Coupling-off groups are described in further detail in: U.S. Patent Nos. 2,355,169; 3,227,551; 3,432,521; 3,476,563; 3,67,291; 3,880,661; 4,052,212 and 4,134,766, and in British Patent Reference Nos. 1,466,788; 1,531,927; 1,533,039; 2,006,755A and 2,017,704A, the disclosures of which are incorporated herein by reference.

Particularly preferred are compounds in which Ar is of the structure: 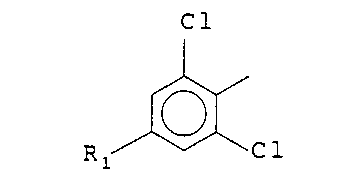 wherein R₁ is selected from the group consisting of halogen, cyano, alkylsulfonyl, arylsulfonyl, sulfamoyl, sulfonamido, carbamoyl, carbonamido, ureido, alkoxycarbonyl, aryloxycarbonyl, acyloxy, alkoxy, aryloxy, nitro and trifluoromethyl groups;
Y is of the structure: 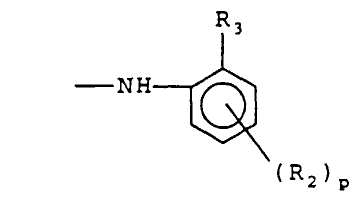 wherein
p is from zero to 2 and each R₂ is in a meta or para position with respect to R₃;
each R₂ is individually selected from the group consisting of halogen, alkyl, alkoxy, aryloxy, carbonamido, carbamoyl, sulfonamido, sulfamoyl, alkylsulfinyl, arylsulfinyl, alkylsulfonyl, arylsulfonyl, alkoxycarbonyl, aryloxycarbonyl, acyloxy, ureido, imido, carbamate, heterocyclic, cyano, nitro, acyl, trifluoromethyl, alkylthio and carboxyl groups; and
R₃ is selected from the group consisting of hydrogen, halogen, alkyl, alkoxy, aryloxy, alkylthio, carbonamido, carbamoyl, sulfonamido, sulfamoyl, alkylsulfonyl, arylsulfonyl, alkoxycarbonyl, acyloxy, acyl, cyano, nitro and trifluoromethyl groups; and
X is of the structure: 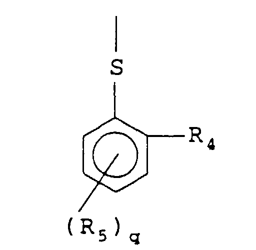 wherein R₄ and R₅ are individually selected from the group consisting of hydrogen, halogen, alkyl, alkoxy, aryloxy, carbonamido, ureido, carbamate, sulfonamido, carbamoyl, sulfamoyl, acyloxy, alkoxycarbonyl, aryloxycarbonyl, amino and carboxyl groups, and wherein q is 0, 1 or 2 and R₅ may be in the meta or para position with respect to the sulfur atom.

Suitable magenta dye-forming couplers for use in the compositions and methods of the present invention include, but are not limited to, the following compounds:

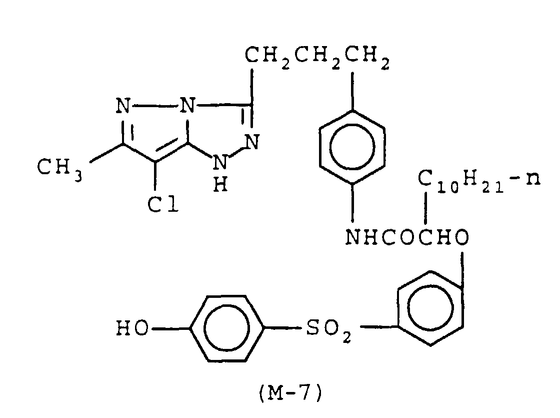

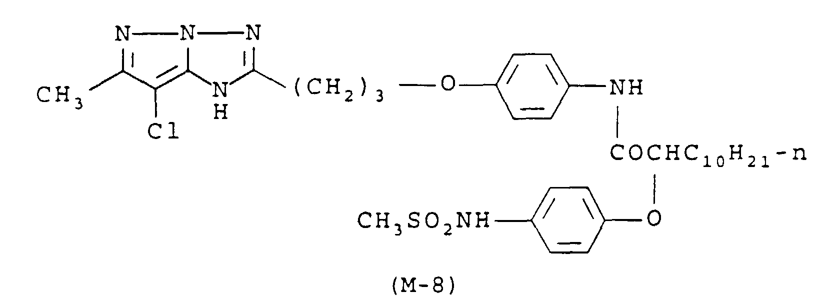

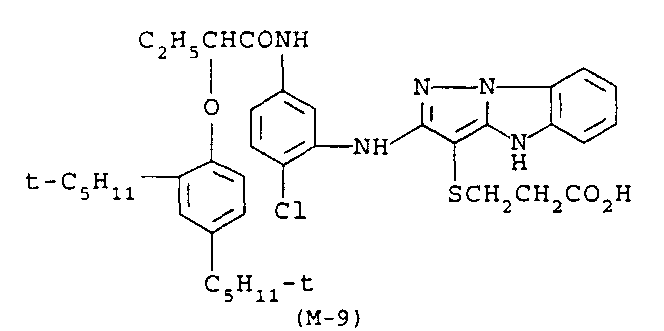

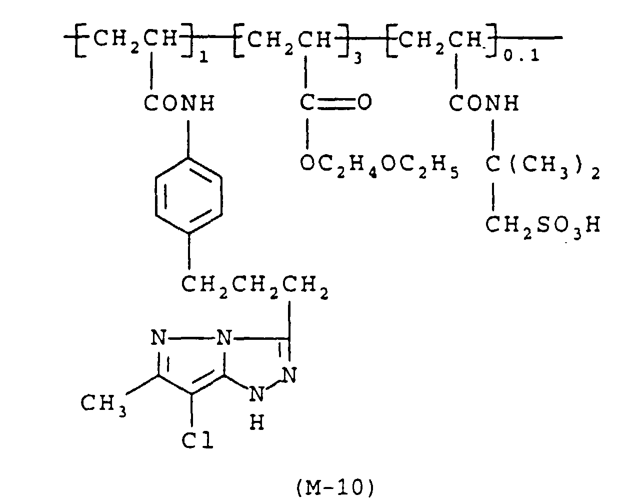

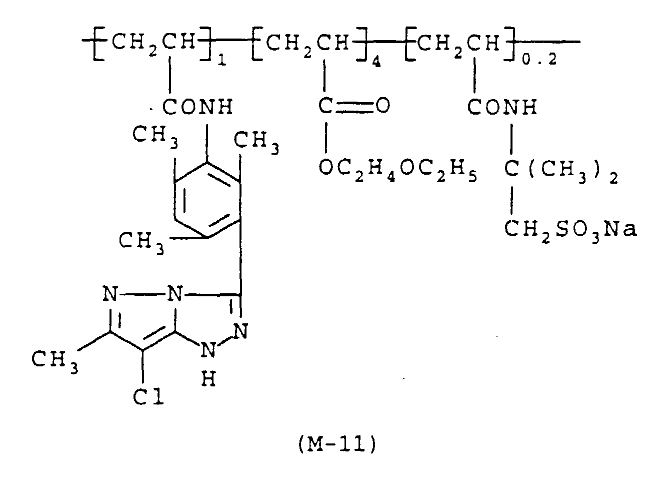

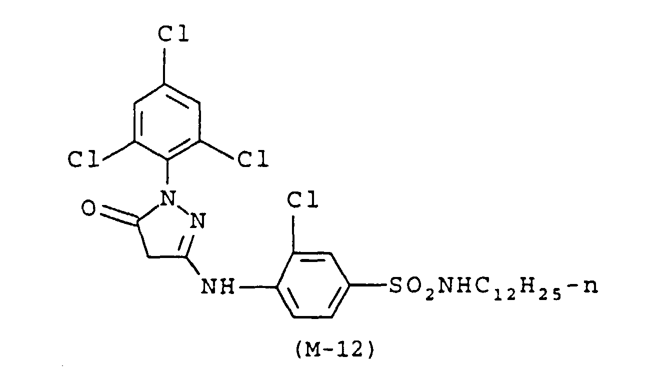

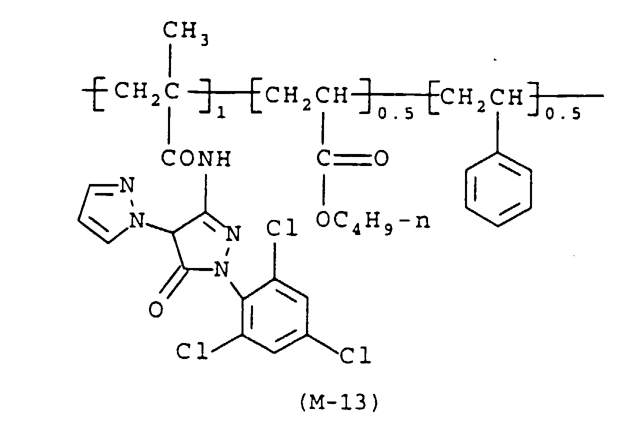

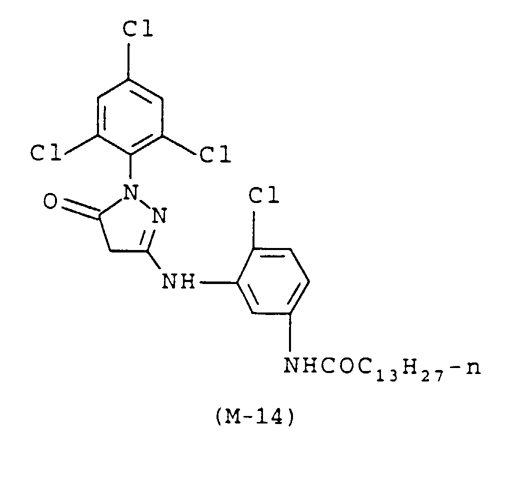

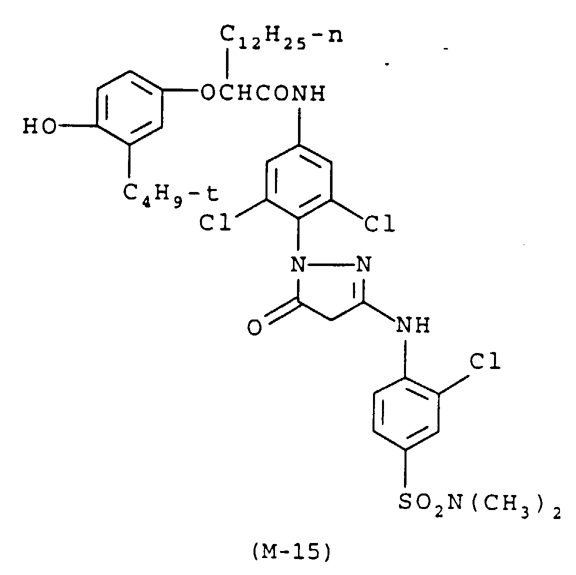

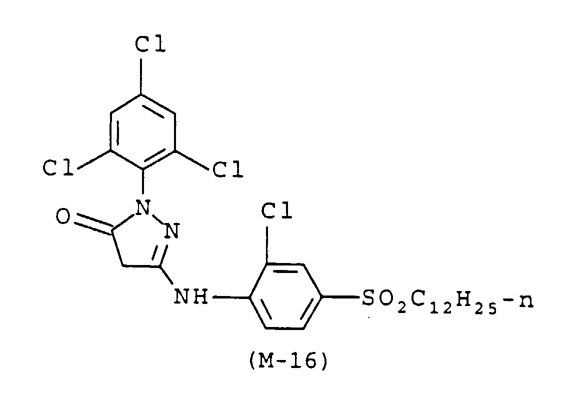

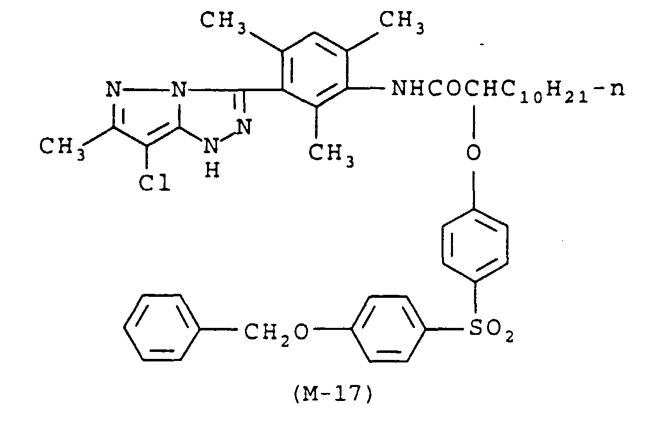

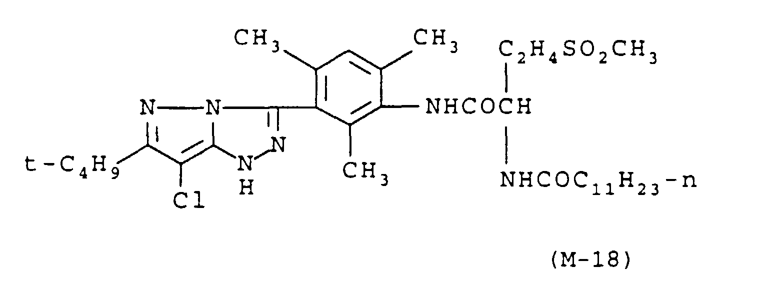

Examples of two-equivalent 3-anilino pyrazolone dye-forming magenta couplers suitable for use in the coupler compositions of the present invention include, but are not limited to the following:

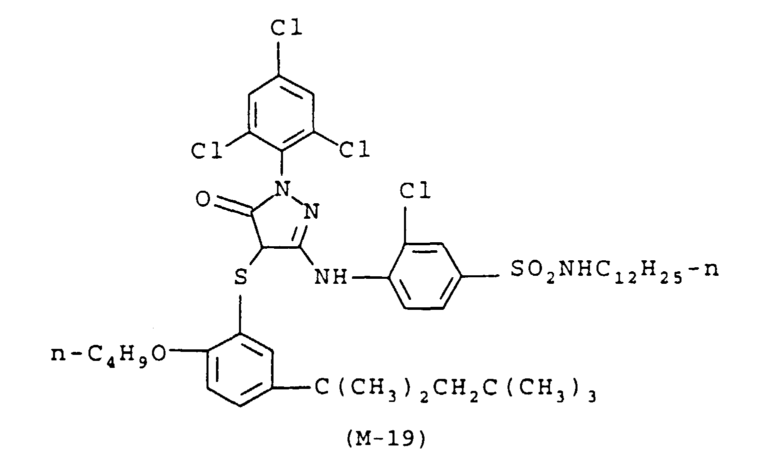

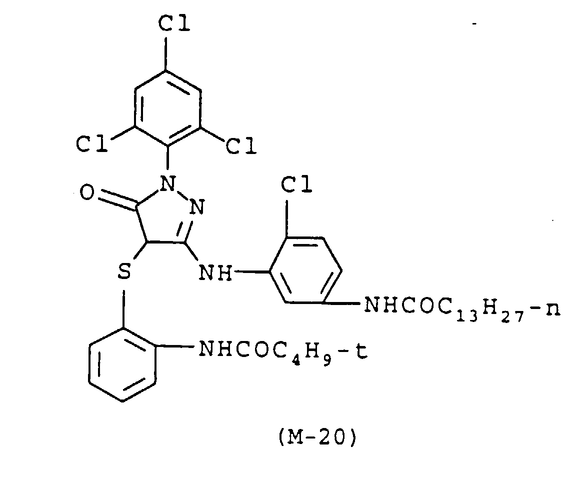

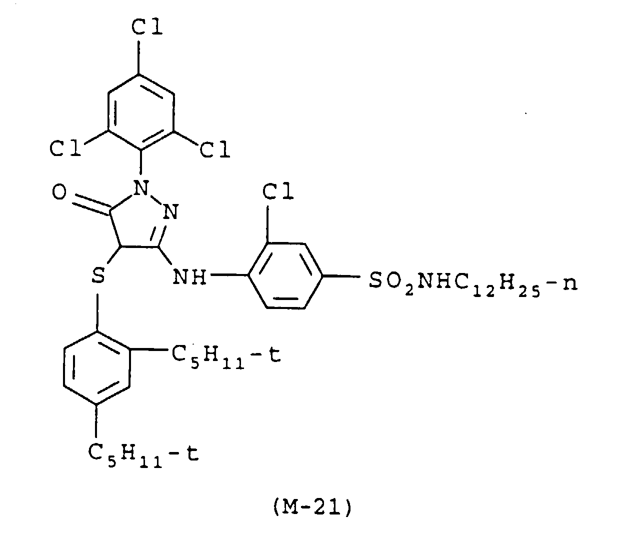

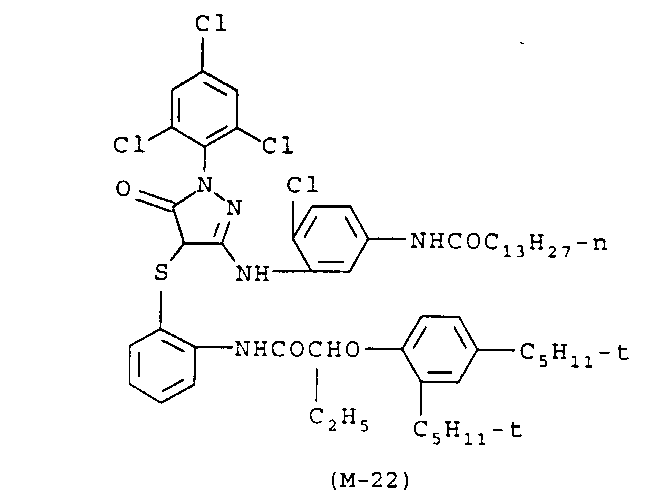

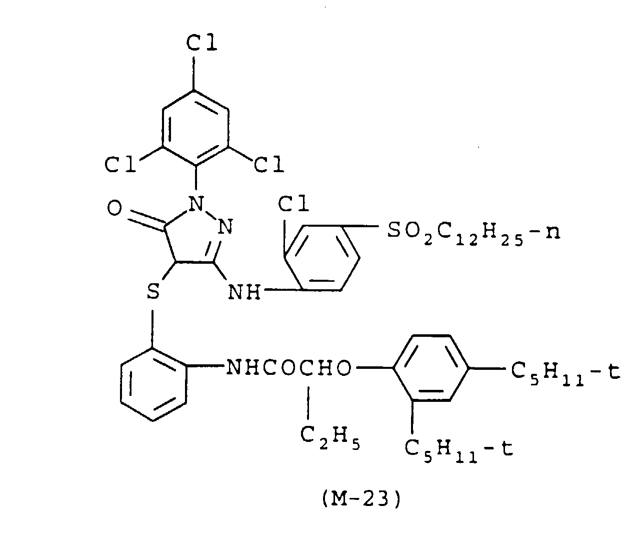

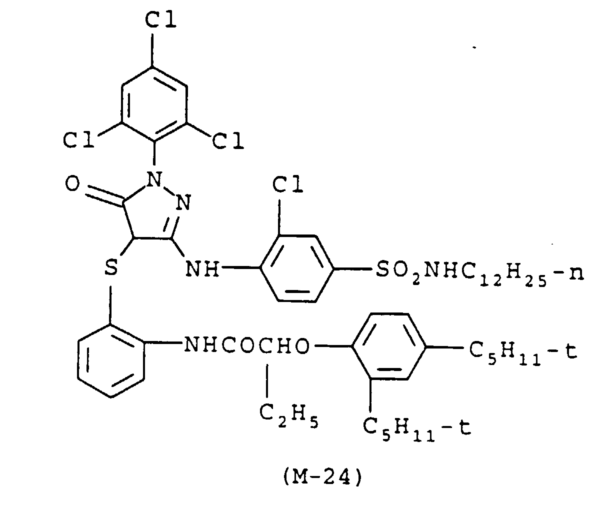

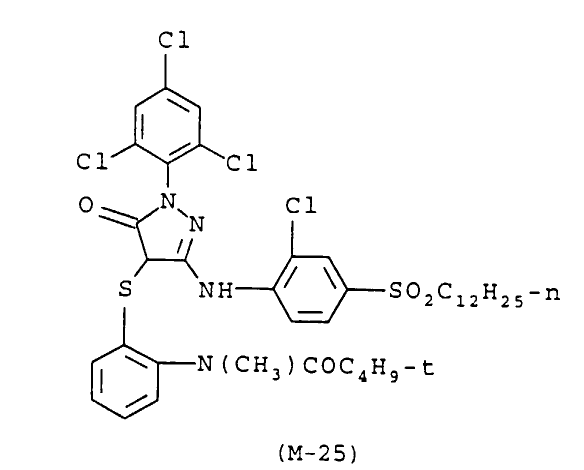

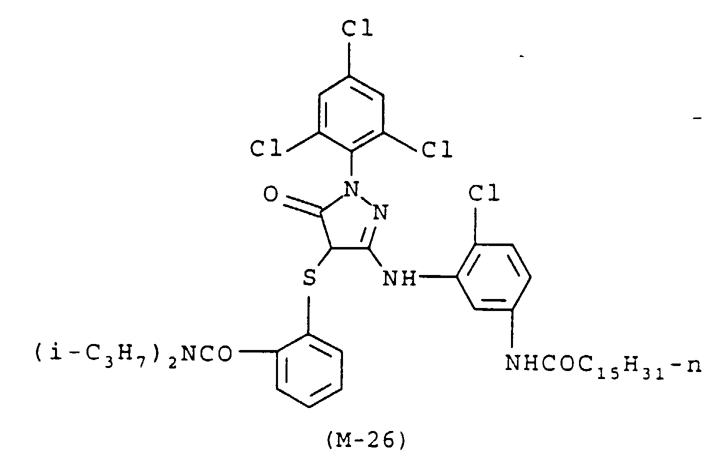

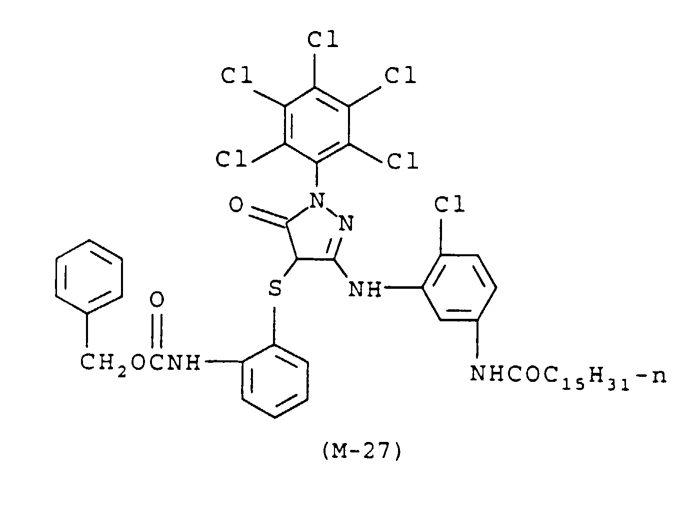

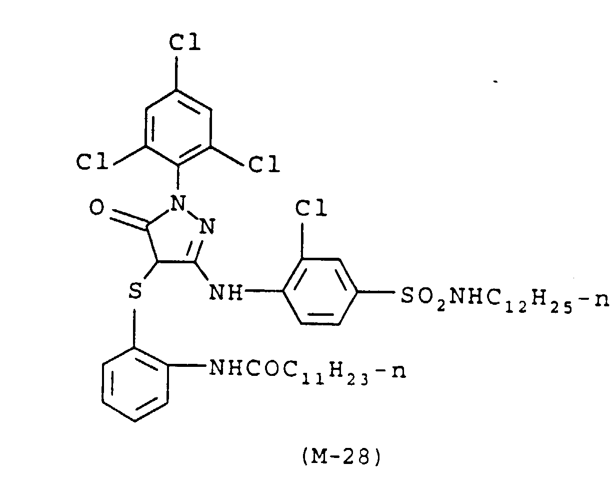

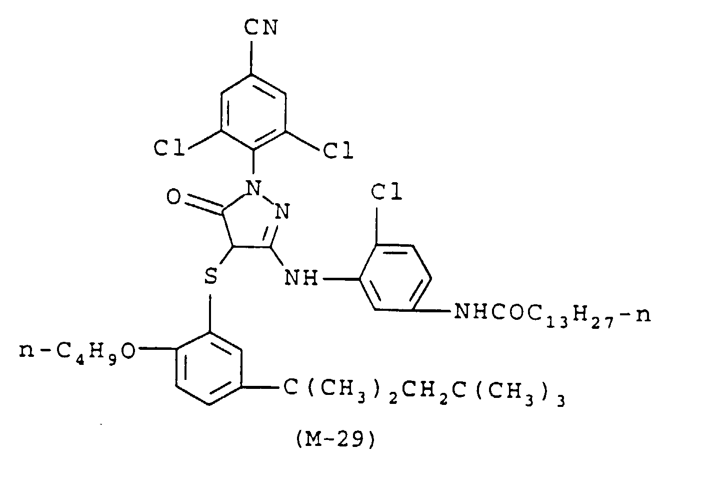

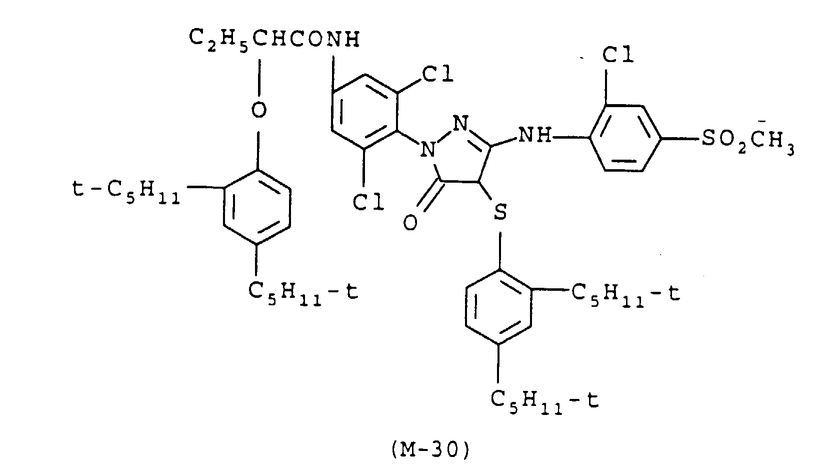

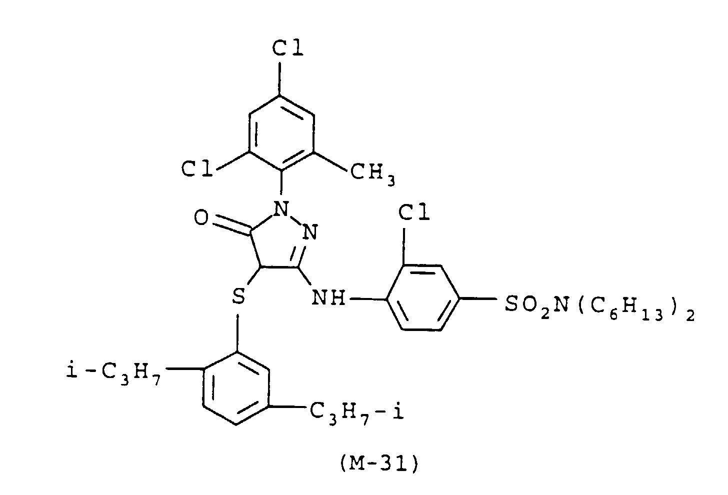

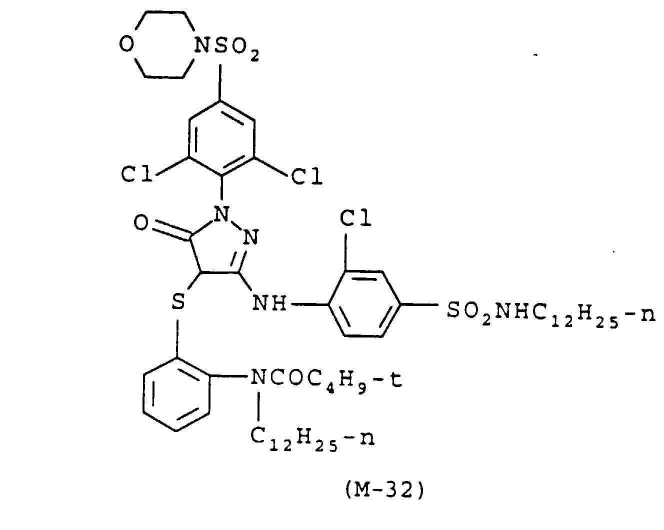

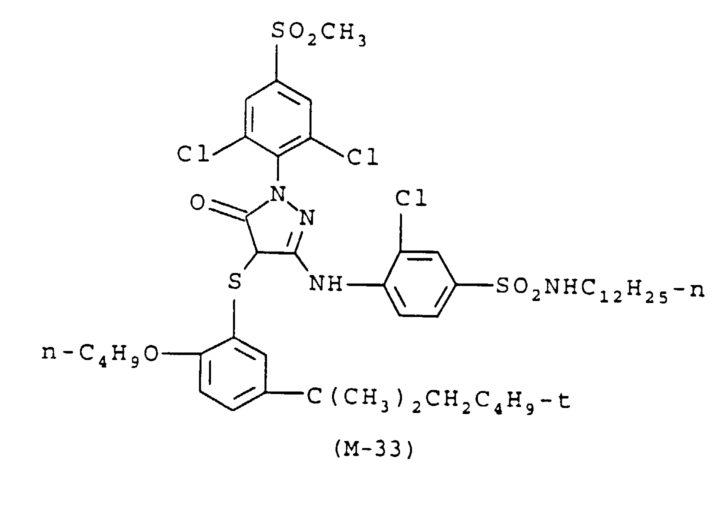

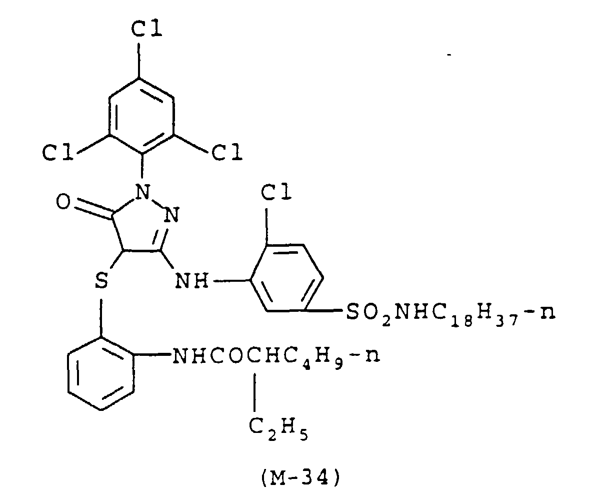

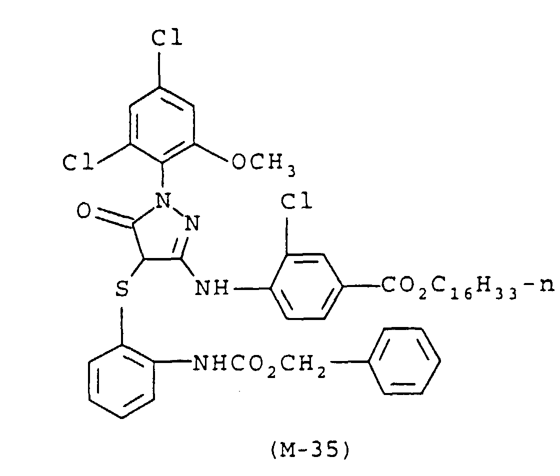

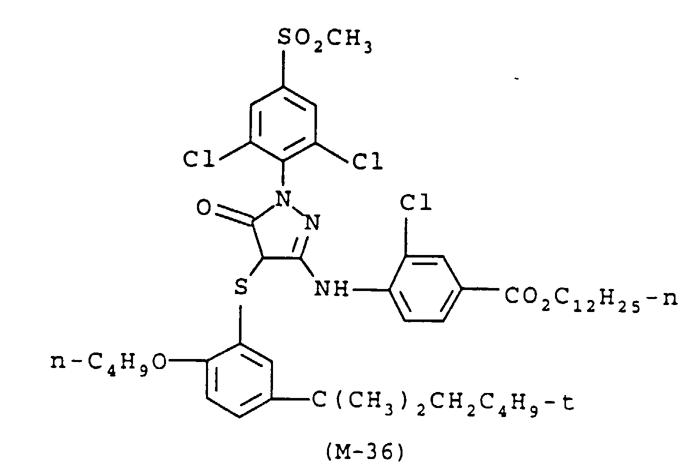

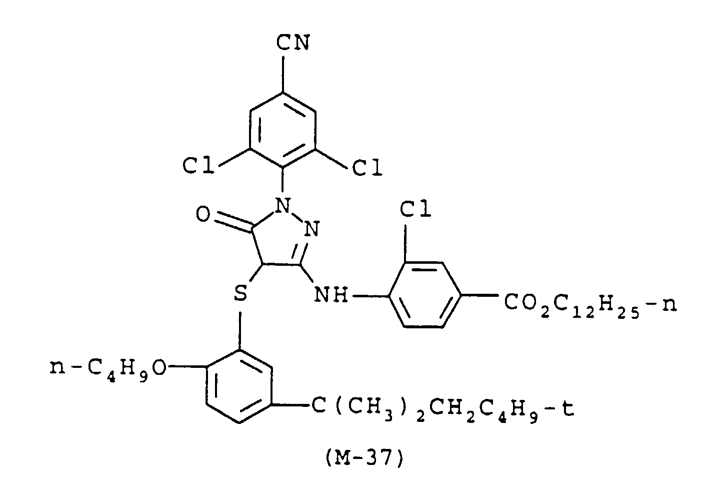

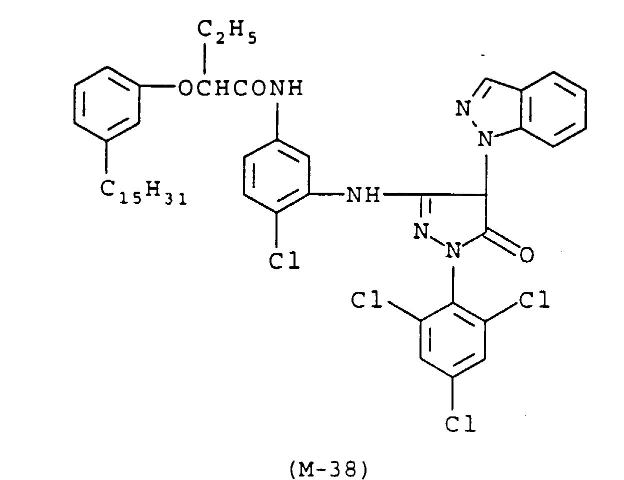

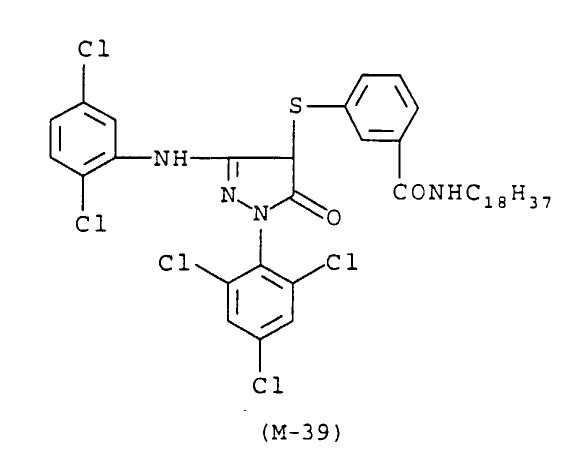

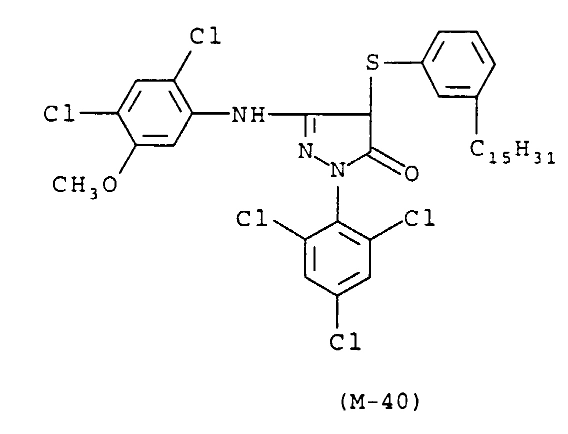

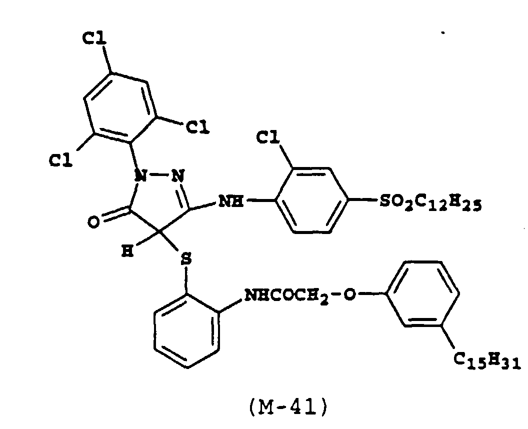

Particularly preferred couplers are compounds of the formulae: M-20, M-21, M-22, M-23, M-24, and M-41.

The color photographic element of this invention comprises, in addition to the magenta coupler-containing layer and the layer comprising the epoxy compound, various other layers typically included in color photographic elements.

Multicolor color photographic elements typically contain image dye-forming units sensitive to each of the three primary regions of the spectrum. Each unit can comprise a single emulsion layer or multiple emulsion layers sensitive to a given region of the spectrum. The layers of the element, including the layers of the image-forming units, can be arranged in various orders as known in the art. In an alternative format, the emulsions sensitive to each of the three primary regions of the spectrum can be disposed as a single segmented layer.

Use of compounds of formula SI in multilayer photographic elements is specifically contemplated. A typical multicolor photographic element comprises a support bearing a cyan dye image-forming unit comprised of at least one red-sensitive silver halide emulsion layer having associated therewith at least one cyan dye-forming coupler, a magenta dye image-forming unit comprising at least one green-sensitive silver halide emulsion layer having associated therewith at least one magenta dye-forming coupler, and a yellow dye image-forming unit comprising at least one blue-sensitive silver halide emulsion layer having associated therewith at least one yellow dye-forming coupler. The element can contain additional layers, such as filter layers, interlayers, overcoat layers, subbing layers, and the like. For example, compounds of formula SI may be used in layers adjacent to the magenta layers of the multilayer elements described in Research Disclosure February 1995, Item 37038, published by Kenneth Mason Publications, Ltd., Dudley Annex, 12a North Street, Emsworth, Hampshire P010 7DQ, ENGLAND. If desired, the photographic elements of the invention can be used in conjunction with an applied magnetic layer as described in Research Disclosure, November 1992, Item 34390.

In the following discussion of suitable materials for use in the photographic element of this invention, reference will be made to Research Disclosure, December 1989, Item 308119, available as described above, which will be identified hereafter by the term "Research Disclosure." The Sections hereafter referred to are Sections of the Research Disclosure.

The silver halide emulsions employed in the elements of this invention can be either negative-working or positive-working. Suitable emulsions and their preparation as well as methods of chemical and spectral sensitization are described in Sections I through IV. Color materials and development modifiers are described in Sections V and XXI. Vehicles are described in Section IX, and various additives such as brighteners, antifoggants, stabilizers, light absorbing and scattering materials, hardeners, coating aids, plasticizers, lubricants and matting agents are described , for example, in Sections V, VI, VIII, X, XI, XII, and XVI. Manufacturing methods are described in Sections XIV and XV, other layers and supports in Sections XIII and XVII, processing methods and agents in Sections XIX and XX, and exposure alternatives in Section XVIII.

The photographic element of this invention generally contains image dye-forming couplers that form cyan dyes upon reaction with oxidized color developing agents which are described in such representative patents and publications as: U.S. Pat. Nos. 2,772,162, 2,895,826, 3,002,836, 3,034,892, 2,474,293, 2,423,730, 2,367,531, 3,041,236, 4,883,746 and "Farbkuppler-eine Literature Ubersicht," published in Agfa Mitteilungen, Band III, pp. 156-175 (1961). Preferably such couplers are phenols and naphthols that form cyan dyes on reaction with oxidized color developing agent.

As discussed above, the photographic element of this invention contains an image dye-forming coupler that forms a magenta dye. Illustrative magenta couplers are set forth above.

The photographic element can also contain couplers that form yellow dyes upon reaction with oxidized and color developing agent are described in such representative patents and publications as: U.S. Pat. Nos. 2,875,057, 2,407,210, 3,265,506, 2,298,443, 3,048,194, 3,447,928 and "Farbkuppler-eine LiteratureUbersicht," published in Agfa Mitteilungen, Band III, pp. 112-126 (1961). Such couplers are typically open chain ketomethylene compounds.

It may be useful to use a combination of couplers any of which may contain known ballasts or coupling-off groups such as those described in U.S. Patent 4,301,235; U.S. Patent 4,853,319 and U.S. Patent 4,351,897. The coupler may also be used in association with "wrong" colored couplers (e.g. to adjust levels of interlayer correction) and, in color negative applications, with masking couplers such as those described in EP 213.490; Japanese Published Application 58-172,647; U.S. Patent 2,983,608; German Application DE 2,706,117C; U.K. Patent 1,530,272; Japanese Application A-113935; U.S. Patents 4,070,191 and 4,273,861; and German Application DE 2,643,965. The masking couplers may be shifted or blocked.

The photographic element can also contain materials that accelerate or otherwise modify the processing steps e.g. of bleaching or fixing to improve the quality of the image. Bleach accelerator releasing couplers such as those described in EP 193,389; EP 301,477; U.S. 4,163,669; U.S. 4,865,956; and U.S. 4,923,784, may be useful. Also contemplated is use of nucleating agents, development accelerators or their precursors (UK Patent 2,097,140; U.K. Patent 2,131,188); electron transfer agents (U.S. 4,859,578; U.S. 4,912,025); antifogging and anti color-mixing agents such as derivatives of hydroquinones, aminophenols, amines, gallic acid; catechol; ascorbic acid; hydrazides; sulfonamidophenols; and non color-forming couplers.

For example, in a color paper format, the photographic element of the invention may comprise a support bearing the following layers from top to bottom:
(1) one or more overcoats;
(2) a cyan layer containing "Coupler 1":
   Butanamide, 2-(2,4-bis(1,1-dimethylpropyl)phenoxy)-N-(3,5-dichloro-2-hydroxy-4-methylphenyl)-, "Coupler 2":
   Acetamide, 2-(2,4-bis(1,1-dimethylpropyl)phenoxy)-N-(3,5-dichloro-2-hydroxy-4-, and UV Stabilizers: Phenol, 2-(5-chloro-2H-benzotriazol-2-yl)-4,6-bis(1,1-dimethylethyl)-;Phenol, 2-(2H-benzotriazol-2-yl)-4-(1,1-dimethylethyl)-;Phenol, 2-(2H-benzotriazol-2-yl)-4-(1,1-dimethylethyl)-6-(1-methylpropyl)-; and Phenol, 2-(2H-benzotriazol-2-yl)-4,6-bis(1,1-dimethylpropyl)-and a poly(t-butylacrylamide) dye stabilizer;
(3) an interlayer;
(4) a magenta layer containing "Coupler 3":
   Octanamide, 2-[2,4-bis(1,1-dimethylpropyl)phenoxy]-N-[2-(7-chloro-6-methyl-1H-pyrazolo[1,5-b] [1,2,4]triazol-2-yl)propyl]- together with 1,1'-Spirobi(1H-indene), 2,2',3,3'-tetrahydro-3,3,3',3'-tetramethyl-5,5',6,6'-tetrapropoxy-;
(5) an interlayer; and
(6) a yellow layer containing "Coupler 4": 1-Imidazolidineacetamide, N-(5-((2-(2,4-bis(1,1-dimethylpropyl)phenoxy)-1-oxobutyl)amino)-2-chlorophenyl)-.alpha.-(2,2-dimethyl-1-oxopropyl)-4-ethoxy-2,5-dioxo-3-(phenylmethyl)-.

The photographic element of the invention can also contain filter dye layers comprising colloidal silver sol or yellow, cyan, and/or magenta filter dyes, either as oil-in-water dispersions, latex dispersions or as solid particle dispersions. Additionally, the photographic element can contain "smearing" couplers (e.g. as described in U.S. 4,366,237; EP 96,570; U.S. 4,420,556; and U.S. 4,543,323.)

The photographic element can also contain image-modifying compounds such as "Developer Inhibitor-Releasing" compounds (DIR's). DIR's useful in conjunction with the compositions of the invention are known in the art and examples are described in U.S. Patent Nos. 3,137,578; 3,148,022; 3,148,062; 3,227,554; 3,384,657; 3,379,529; 3,615,506; 3,617,291; 3,620,746; 3,701,783; 3,733,201; 4,049,455; 4,095,984; 4,126,459; 4,149,886; 4,150,228; 4,211,562; 4,248,962; 4,259,437; 4,362,878; 4,409,323; 4,477,563; 4,782,012; 4,962,018; 4,500,634; 4,579,816; 4,607,004; 4,618,571; 4,678,739; 4,746,600; 4,746,601; 4,791,049; 4,857,447; 4,865,959; 4,880,342; 4,886,736; 4,937,179; 4,946,767; 4,948,716; 4,952,485; 4,956,269; 4,959,299; 4,966,835; 4,985,336 as well as in patent publications GB 1,560,240; GB 2,007,662; GB 2,032,914; GB 2,099,167; DE 2,842,063, DE 2,937,127; DE 3,636,824; DE 3,644,416 as well as the following European Patent Publications: 272,573; 335,319; 336,411; 346, 899; 362, 870; 365,252; 365,346; 373,382; 376,212; 377,463; 378,236; 384,670; 396,486; 401,612; 401,613. Such compounds are also disclosed in "Developer-Inhibitor-Releasing (DIR) Couplers for Color Photography," C.R. Barr, J.R. Thirtle and P.W. Vittum in Photographic Science and Engineering, Vol. 13, p. 174 (1969)

It is also contemplated that the concepts of the present invention may be employed to obtain reflection color prints as described in Research Disclosure, November 1979, Item 18716, available from Kenneth Mason Publications, Ltd, Dudley Annex, 12a North Street, Emsworth, Hampshire P0101 7DQ, England. Other compounds that can be included in the photographic element of this invention are disclosed in Japanese Published Applications described in Derwent Abstracts having accession numbers as follows: 90-072,629, 90-072,630; 90-072,631; 90-072,632; 90-072,633; 90-072,634; 90-077,822; 90-078,229; 90-078,230; 90-079,336; 90-079,337; 90-079,338; 90-079,690; 90-079,691; 90-080,487; 90-080,488; 90-080,489; 90-080,490; 90-080,491; 90-080,492; 90-080,494; 90-085,928; 90-086,669; 90-086,670; 90-087,360; 90-087,361; 90-087,362; 90-087,363; 90-087,364; 90-088,097; 90-093,662; 90-093,663; 90-093,664; 90-093,665; 90-093,666; 90-093,668; 90-094,055; 90-094,056; 90-103,409; 83-62,586; 83-09,959.

Especially useful in this invention are tabular grain silver halide emulsions. Specifically contemplated tabular grain emulsions are those in which greater than 50 percent of the total projected area of the emulsion grains are accounted for by tabular grains having a thickness of less than 0.3 µm (0.5 µm for blue sensitive emulsion) and an average tabularity (T) of greater than 25 (preferably greater than 100), where the term "tabularity" is employed in its art recognized usage as T = ECD/t² where ECD is the average equivalent circular diameter of the tabular grains in microns and t is the average thickness in microns of the tabular grains.

Suitable tabular grain emulsions can be selected from among a variety of conventional teachings, such as those of the following: Research Disclosure, Item 22534, January 1983, published by Kenneth Mason Publications, Ltd., Emsworth, Hampshire P010 7DD, England; U.S. Patent Nos. 4,439,520; 4,414,310; 4,433,048; 4,643,966; 4,647,528; 4,665,012; 4,672,027; 4,678,745; 4,693,964; 4,713,320; 4,722,886; 4,755,456; 4,775,617; 4,797,354; 4,801,522; 4,806,461; 4,835,095; 4,853,322; 4,914,014; 4,962,015; 4,985,350; 5,061,069 and 5,061,616. In addition, use of [100] silver chloride emulsions as described in EP 534,395 are specifically contemplated.

The emulsions can be surface-sensitive emulsions, i.e., emulsions that form latent images primarily on the surfaces of the silver halide grains, or the emulsions can form internal latent images predominantly in the interior of the silver halide grains. The emulsions can be negative-working emulsions, such as surface-sensitive emulsions or unfogged internal latent image-forming emulsions, or direct-positive emulsions of the unfogged, internal latent image-forming type, which are positive-working when development is conducted with uniform light exposure or in the presence of a nucleating agent.

Photographic elements can be exposed to actinic radiation, typically in the visible region of the spectrum, to form a latent image and can then be processed to form a visible dye image. Processing to form a visible dye image includes the step of contacting the element with a color developing agent to reduce developable silver halide and oxidize the color developing agent. Oxidized color developing agent in turn reacts with the coupler to yield a dye.

The photographic elements can be processed, for example, in accordance with color print processes such a the RA-4 process of Eastman Kodak Company as described in the British Journal of Photography Annual of 1988, Pp 198-199.

Preferred color developing agents are p-phenylenediamines such as: 4-amino-N,N-diethylaniline hydrochloride, 4-amino-3-methyl-N,N-diethylaniline hydrochloride, 4-amino-3-methyl-N-ethyl-N-(b-(methanesulfonamido) ethyl)aniline sesquisulfate hydrate, 4-amino-3-methyl-N-ethyl-N-(b-hydroxyethyl)aniline sulfate, 4-amino-3-b-(methanesulfonamido)ethyl-N,N-diethylaniline hydrochloride and 4-amino-N-ethyl-N-(2-methoxyethyl)-m-toluidine di-p-toluene sulfonic acid.

Development is usually followed by the conventional steps of bleaching, fixing, or bleach-fixing, to remove silver or silver halide, washing, and drying.

The following examples illustrate the invention:

### Example 1

### Synthesis of SI-19:

### Reaction of p-Aminophenol with 2-Mesitylenesulfonyl Chloride. Synthesis of 4A.

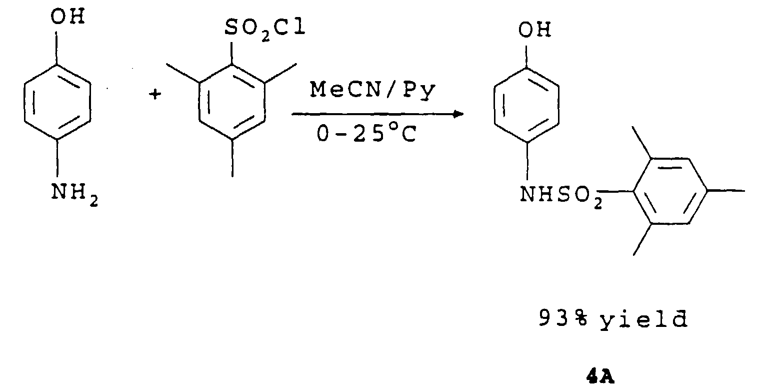

A 1L flask equipped with a magnetic stirring bar and a pressure equalized addition funnel was charged with p-aminophenol (12.47g, 114.31 mmol), and MeCN (200mL). This solution was chilled in a ice/water bath for 10 minutes while stirring an Argon atmosphere. To this cold tan solution pyridine (9.22g, 116.60 mmol) was added and the let stirred for 20 minutes. A pressure equalized addition funnel was charged with 2-mesitylenesulfonyl chloride (25.0g, 114.31 mmol) with 90mL of MeCN. The sulfonyl chloride solution was then added dropwise over 30 minutes. A color change from red to amber was observed during addition; the formation of a white precipitate was also noted. The reaction was stirred at room temperature until reaction was complete by tlc analysis (16 hours). Reaction mixture was poured into ice water (500mL) and partitioned with EtOAc (200mL), and transferred to separatory funnel. Layers were separated and aqueous layer was extracted with EtOAc (2 x 100mL), organic layers were combined, washed with Sat. NH₄Cl (1 x 250mL), brine (1 x 250mL), dried over MgSO₄, filtered and stripped to give an off white solid (32.77g). Crude product was recrystallized from EtOAc/hexanes to give two crops of 4A, as a white crystalline solid.mp 189-190°C, (30.95g, 93%) ¹HNMR (300 MHz) DMSO; δ 2.16 (s, 3H), 2.40 (s, 6H), (d, J=8.4 Hz, 2H), 6.72 (d, J=8.4 Hz, 2H), 6.90 (s, 2H), 9.28 (s, 1H), 9.47 (s, 1H). Anal. Calcd for C₁₅H₁₇NO₃S: C, 61.83; H, 5.88; N; 4.81. Found C, 61.51; H, 5.96; N, 4.73.

### Reaction of p-(2-Mesitylenesulfonamido)-Phenol with Undeceonyl Chloride. Synthesis of 4B.

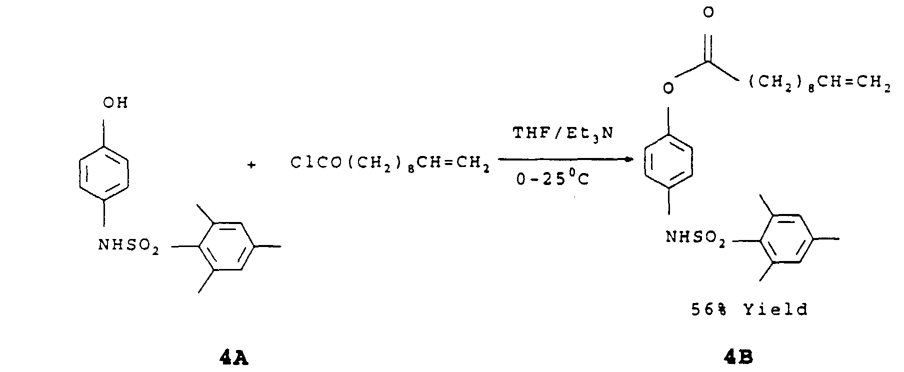

A 1L flask equipped with magnetic stirring bar and a pressure equalized addition funnel was charged with 4A (29.10g, 99.87 mmol), THF (300mL), and Et₃N (14.6mL, 101.87 mmol). This solution was chilled in an ice/water bath for 10 minutes while stirring under an Argon atmosphere. A pressure equalized addition funnel was charged with undecenolyl chloride (20.45g, 100.87 mmol). The acid chloride was then added dropwise over 1-2 hours to the THF solution. A white precipitate formed on addition (Et₃N:HCl). The reaction took on a pale green color shortly after acid chloride addition was begun; this color persisted for several hours. Reaction was allowed to stir for 16 hours warming to room temperature. Tlc analysis (10:1 ligroin 950/EtOAc) showed two new spots (Rf = 0.55 and 0.25) and starting material(Rf = 0.08). Addition of 0.5 Eq of acid chloride after prolonged stirring (72 hours)at room temperature showed no change by tlc analysis. Reaction mixture was poured into ice water (750 mL) and partitioned with EtOAc (300mL), and transferred to separatory funnel. Layers were separated and aqueous layer was extracted with EtOAc (2 x 300mL), organic layers were combined, washed with Sat. NaHCO₃ (1 x 250 mL), brine (1 x 250 mL), dried over MgSO4, filtered and stripped to give an amber oil (47g). Crude product was chromatographed over silica gel (400-600µ) and eluted with 10:1 ligroin 950/EtOAc. Fractions were pooled and solvent stripped to give the desired product (4B), as a clear oil (25.6g, 56%). FDMS m/e = 457. ¹HNMR (300 MHZ) CDCl₃: δ 1.35 (m, 10H), 6.91 (m, 6H), 7.16 (s, 1H). Anal. Calcd for C₂₆H₃₅NO₄S: C, 68.24; H, 7.71; N; 3.06. Found: C, 68.60; H, 7.65; N, 2.90.

### Reaction of p-(2-mesitylenesulfonamido)-Phenyl Undecenoate with m-Chloroperbenzoic acid. Synthesis of SI-19.

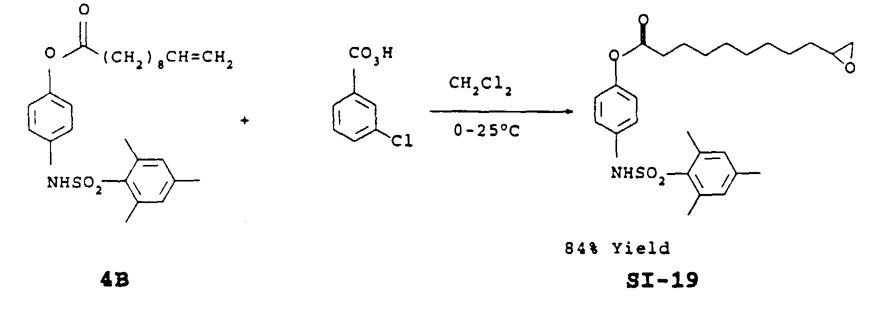

A 500mL flask equipped with a magnetic stirring bar and a pressure equalized addition funnel was charged with 4B (24.50g, 53.53 mmol), CH₂Cl₂ (150 mL). This solution was placed in a room temperature water bath. A pressure equalized addition funnel was charged with a solution of m-perbenzoic acid (80%, tech. grade; 17.30g, 80.30 mmol) in CH₂Cl₂ (150 mL)¹. The m-CPBA solution was then added dropwise over 1 hour and the reaction let stir for 72 hours at room temperature, during this time a white precipitate had formed (CBA). Tlc analysis (5:1 ligroin 950/EtOAc) showed complete conversion of starting material. White solids were filtered off and discarded; tlc analysis matched authentic sample of CBA. Filtrate was transferred to a separatory funnel and treated with a solution of 10% Na₂SO₃ (150 ml), separated layers, aqueous layer was discarded, and organic layer was washed with 10% NaHCO₃ (1 x 100mL), brine (1 x 100mL), and dried (MgSO₄), filtered and stripped to give a pale yellow oil, which on cooling formed a pale yellow solid (32.4g).
Recrystallization of crude product from EtOAc/Hexanes with chilling in ice gave two crops of SI-19, as an off white crystalline solid, which was dried at 50°C overnight in a vacuum oven (25 in. Hg), to give 21.32g (84%) of an off white crystalline powder, mp. 77-78°C. FDMS m/e - 474 (MH⁺). ¹HNMR (300 MHZ) CDCl₃:δ1.39 (m, 10H), 1.43 (m, 2H) 1.68 (m, 2H), 2.24 (s, 3H), 2.46 (m, 3H), 2.56 (s, 6H), 2.74 (m, 1H), 2.90 (br.s, 1H), 6.90 (m, 6H), 7.08 (s, 1H). Anal. Calcd for C₂₆H₃₅NO₅S: C, 65:93, H, 7.45; N; 2.96. Found: C, 66.03; H, 7.42; N, 2.66.
¹ The total volume of the reaction mixture was chosen to afford a [m-CPBA] = 0.33M, at this concentration the CBA byproduct of the reaction precipitates and may be removed by filtration.

### Example 2

### Synthesis of SI-20:

### Reaction of p-Aminophenol with 2,4-diflurobenzene Sulfonyl Chloride. Synthesis of 5A.

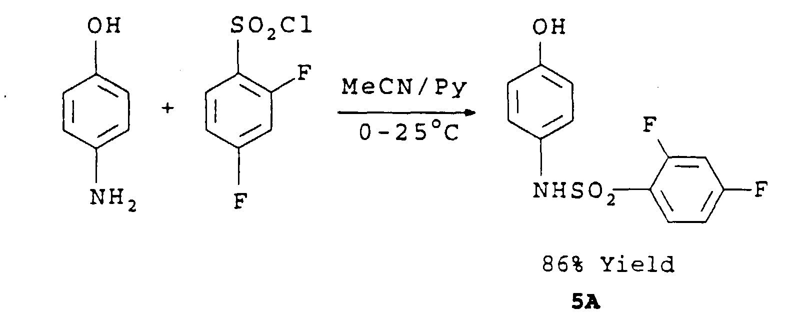

A 1L flask equipped with a magnetic stirring bar and a pressure equalized addition funnel was charged with p-aminophenol (12.83g., 117.59 mmol), and MeCN (200mL). This solution was chilled in a ice/water bath for 10 minutes while stirring under an Argon atmosphere. To this cold tan solution pyridine (9.49g., 119.94 mmol) was added and the let stir for 20 minutes. A pressure equalized addition funnel was charged with 2,4-difluorobenzene sulfonyl chloride (25.0g, 117.59 mmol) with 10mL of MeCN. The sulfonyl chloride solution was then added dropwise over 30 minutes. A color change from red to red/orange was observed during addition. The reaction was stirred warming to room temperature until reaction was complete by tlc analysis. Reaction mixture was poured into ice water (500mL) and partitioned with EtOAc (200 mL), and transferred to separatory funnel. Layers were separated and aqueous layer was extracted with EtOAc (2 x 100mL), organic layers were combined, washed with Sat. NH₄Cl (1 x 250mL), brine (1 x 250 mL), dried over MgSO4, filtered and stripped to give a pink solid (32.17g). Crude product was dissolved in a minimum of EtOAc and passed through a plug of silica gel (400-600m) and eluted with 2:1/Ligroin 950/EtOAc, elutent was stripped to give a white solid, which was immediately recrystallized from EtOAc/hexanes to give several crops of 5A, as a white crystalline solid.mp 146-148°C, (23.17g, 86%). ¹HNMR (300 MHz) DMSO: δ 6.58 (d, J=8.6 Hz, 2H), 6.82 (d, J=8.6 Hz, 2H), 7.15 (t, J=8.6 Hz, 1H), 7.47 (t, J=11.2 Hz, 1H), 7.69 (q, J=8.6 Hz, 1H), 9.34 (br.s, 1H), 10.06 (br.s, 1H).

### Reaction of p(2, 4-Diflurobenzene Sulfonamido)-Phenol with Undecenoyl Chloride. Synthesis of 5B.

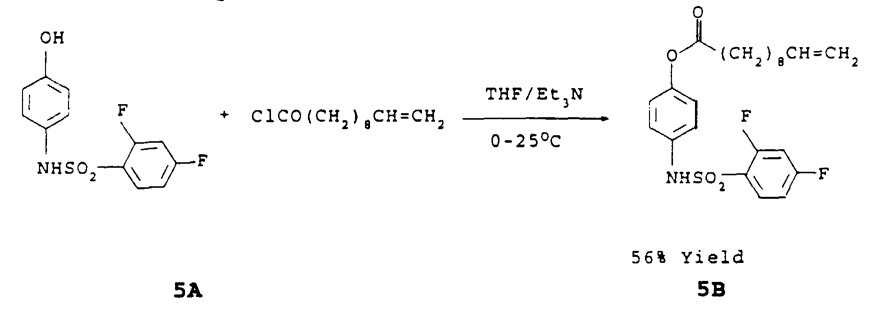

A 1L flask equipped with a magnetic stirring bar and a pressure equalized addition funnel was charged with 5A (27.81 g, 97.49 mmol), THF (350 mL), and Et₃N (14.3 mL, 99.44 mmol). This solution was chilled in a ice/water bath for 10 minutes while stirring under an Argon atmosphere. A pressure equalized addition funnel was charged with undecenoyl chloride (19.96g, 98.46 mmol). The acid chloride was then added dropwise over 30-45 minutes to the THF solution. A white precipitate formed on addition (Et₃N:HCl). The reaction color changed from tan to blue shortly after acid chloride addition was begun. Reaction was allowed to stir for 16 hours warming to room temperature. Tlc analysis (10:1 ligroin 950/EtOAc) showed two new spots (Rf = 0.50 and 0.25) and starting material (Rf = 0.08). Addition of 0.5 Eq of acid chloride after prolonged stirring (72 hours) at room temperature showed only a trace of starting material by tlc analysis. Reaction mixture was poured into ice water (550 mL) and partitioned with EtOAc (300mL), and transferred to separatory funnel. Layers were separated and aqueous layer was extracted with EtOAc (2 x 300 mL), organic layers were combined, washed with Sat. NaHCO₃ (1 x 250 mL), brine (1 x 250 mL) dried over MgSO₄, filtered and stripped to give an amber oil (45g). Crude product was chromatographed over silica gel (400-600m) and eluted with 10:1 ligroin 950/EtOAc. Fractions were pooled and solvent stripped to give: the desired product (5B), as a clear oil which upon trituration crystallized as a white solid mp. 68-72°C (18.40g, 56%). FDMS m/e=451. ¹HNMR (300 MHz) CDCl₃: δ 1.35 (m, 12H), 1.69 (m, 2H), 2.02 (m, 2H), 2.49 (t, J=7.4 Hz, 2H), 4.94 (m, 2H), 5.78 (m, 1H), 6.92 (m, 4H), 7.03 (s, 1H), 7.08 (s, 1H), 7.11 (s, 1H), 7.80 (q, J=8.0 Hz, 1H). Anal. Calcd for C₂₃H₂₇F₂NO₄S: C, 61:18; H, 6.03; N; 3.10. Found C, 60.89; H, 6.08; N, 3.03.

### Reaction of p-(2,4-difluorobenzene Sulfonamido)-Phenyl Undecenoate with m-Chloroperbenzoic acid. Synthesis of SI-20.

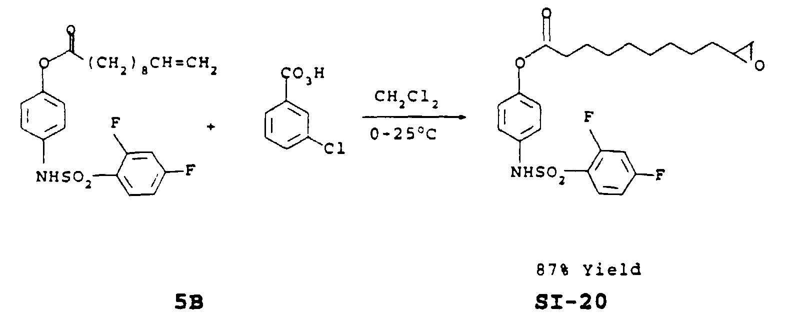

A 500mL flask equipped with a magnetic stirring bar and a pressure equalized addition funnel was charged with 5B (21.82g, 48.32 mmol), CH₂Cl₂ (100mL). This solution was placed in a room temperature water bath. A pressure equalized addition funnel was charged with a solution of m-perbenzoic acid (80%, tech. grade; 15.60g., 72.49 mmol) in CH₂Cl₂ (150 mL)(see footnote page 55). The m-CPBA solution was then added dropwise over 1 hour and the reaction let stir for 72 hours at room temperature, during this time a white precipitate had formed (CBA). Tlc analysis (5:1 ligroin 950/EtOAc) showed complete conversion of starting material. White solids were filtered off and discarded; tlc analysis matched authentic sample of CBA. Filtrate was transferred to a separatory funnel and treated with a solution of 10% Na₂SO₃ (120 ml), separated layers, aqueous layer was discarded, and organic layer was washed with 10% NaHCO₃ (1 x 100mL), brine (1 x 100mL), and dried (MgSO₄), filtered and stripped to give a pale yellow oil, which on cooling formed a pale yellow waxy solid (24.2g). Recrystallization of crude product from EtOAc/Hexanes with chilling in ice gave two crops of SI-20, as a white crystalline solid, which was dried at 60°C overnight in a vacuum oven (25 in Hg), to give 19.74g (87%) of a white crystalline powder.
mp.77°C(s). FDMS m/e = 467. ¹H NMR (300 MHz)CDCl₃: δ 1.45 (m,10H), 1.52 (m, 2H), 1.69 (m, 2H), 2.49 (m, 3H), 2.75 (m, 1H), 2.91 (br.s, 1H) 6.87 (s, 1H), 6.91 (m, 3H), 7.09 (d,J=8.6 Hz, 2H), 7.46 (s,1H), 7.81 (q,J=7.6 Hz), 1H). ¹⁹F NMR (300 MHz, ref. TFT) DMSO: δ-100.5 (s, 1F), -106.2 (s, 1F). Anal. Calcd for C₂₃H₂₇F₂NO₅S: C, 59.09; H, 5.82; N' 3.00. Found C, 58.77; H, 5.85; N, 2.98.

### Example 3

### Synthesis of SI-2:

### Reaction of p-n-Butylsulfonamido-Phenol with Undecenoyl Chloride. Synthesis of 2B.

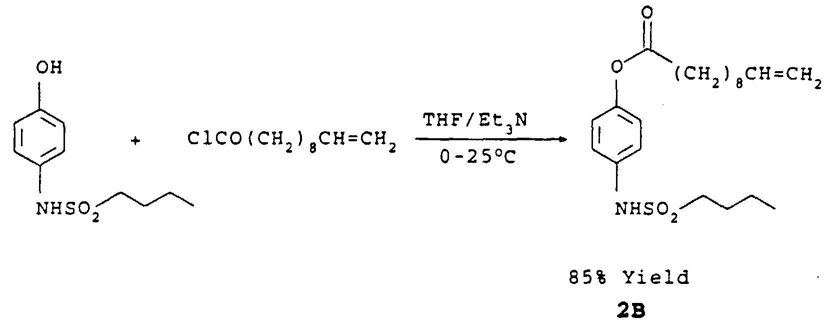

A 500mL flask equipped with a magnetic stirring bar and a pressure equalized addition funnel was charged with 2A (18.02g, 78.59 mmol), THF (225mL), and Et₃N (11.5 mL, 80.16 mmol). A pressure equalized addition funnel was charged with undecenoyl chloride (15.95g, 78.59 mmol). The acid chloride was then added dropwise over 30-45 minutes to the THF solution at room temperature. A white precipitate formed an addition (Et₃N:HCL). Tlc analysis after 2 hours (10:1 ligroin 950/EtOAc) showed complete conversion of starting material to one product. The reaction mixture was poured into ice water (600 mL) and conversion of starting material to one product. The reaction mixture was poured into ice water (660 mL) and partitioned with EtOAc (200mL), and transferred to separatory funnel. Layers were separated and aqueous layer was extracted with EtOAc (2 x 200mL), organic layers were combined, washed with Sat. NaHCO₃ (1 x 250 mL), brine (1 x 250 mL), dried over MgSO₄, filtered and stripped to give an amber oil, which on cooling formed a white solid (34g). Crude product was recrystallized from hexanes/EtOAc, chilled to give 2B, as a white crystalline solid mp. 90-91°C. (26.43g, 85%). FDMS m/e=395. ¹HNMR (300 MHz) CDCl₃: δ 0.89 (t, J=7.3 Hz, 3H), 1.38 (m, 12H), 1.77 (m, 4H), 2.04 (q, J=6.5 Hz, 2H), 4.95 (m, 2H), 5.78 (m, 1H) 6.85 (s, 1H), 7.04 (d, J=8.8 Hz, 2H), 7.21 (d, J=8.8 Hz, 2H). Anal. Calcd. for C₂₁H₃₃NO₄S: C, 63.77; H, 8.41; N; 3.54. Found: C, 63.21; H, 8.06; N, 3.79.

### Reaction of p-(n-Butylsulfonamido)-Phenyl Undecenoate with m-Chloroperbenzoic acid. Synthesis of SI-2.

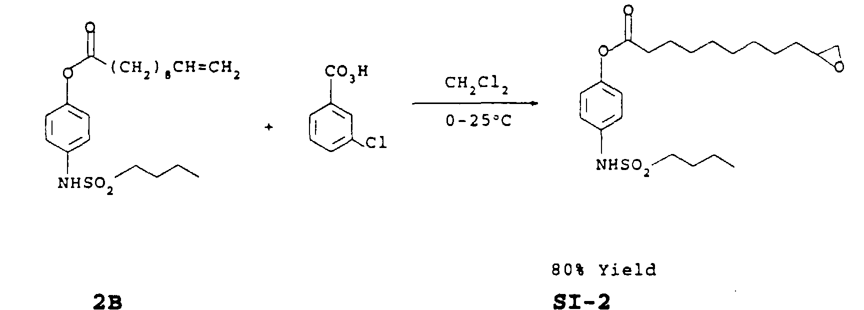

A 500mL flask equipped with a magnetic stirring bar and a pressure equalized addition funnel was charged with a solution of m-perbenzoic acid (80%, tech. grade; 32.70g. 151.55 mmol) in CH₂Cl₂ (320mL) (see footnote page 55). The m-CPBA solution was then added dropwise over 2 hours and the reaction let stir for 16 hours at room temperature, during this time a white precipitate had formed (CBA) Tlc analysis (5:1 ligroin 950/EtOAc) showed complete conversion of starting material. White solids were filtered off and discarded; tlc analysis matched authentic sample of CBA. Filtrate was transferred to a separatory funnel and treated with a solution of 10% Na₂SO₃ (150 ml), separated layers, aqueous layer was discarded, and organic layer was washed with 10% NaHCO₃ (1 x 200 mL), brine (1 x 200 mL), and dried (MgSO₄), filtered and stripped to give a yellow oil, which on cooling formed a pale yellow waxy solid (42.3g). Recrystallization of crude product from EtOAc/Hexanes with chilling in ice gave two crops of SI-2, as a white crystalline solid, which was dried at 60°C overnight in a vacuum oven (25 in Hg), to give 33.20g (80%) of a white crystalline powder. mp. 69-70°C(s). FDMS m/e = 411. ¹HNMR (300 MHz) CDCl₃: δ 0.88 (t, J=7.3 Hz, 3H), 1.35 (m, 12H), 1.46 (m, 2H), 1.77 (m, 4H), 2.46 (m, 1H) 2.53 (t, J=7.5 Hz, 2H), 2.74 (m, 1H), 2.87 (br.s, 1H), 3.06 (t, J=8.1 Hz, 2H), 6.93 (s, 1H), 7.02 (d, J=8.8 Hz, 2H), 7.21 (d, J-8.8 Hz, 2H). Anal. Calcd for C₂₁H₃₃NO₅S: C, 61.29; H, 8.08; N; 3.40. Found: C, 61.01; H, 7.94; N, 3.37.

### Example 4 (Comparative)

A dispersion of the magenta coupler M-20 and scavenger compound S-1 were prepared in the following manner:

### Dispersion of M-20

The oil phase was prepared by combining 11.55 grams M-20 with 11.55 grams of tri(2-ethylhexyl) phosphate coupler solvent and 78.1 grams of ethyl acetate. The solution was stirred for about 10 minutes.

The aqueous phase was prepared by combining 19.2 grams of 10% solution of Alkanol XC with 169.5 grams of 11.36% solution of Type IV gelatin in water. 173.2 grams of distilled water was then added and the solution was stirred on a hot plate for about five minutes.

The aqueous phase was combined with the oil phase and the mixture was passed three times through a colloid mill to obtain the dispersion. The ethyl acetate was then removed by evaporation under reduced pressure.

### Dispersion of S-1

The oil phase was prepared by combining 1.875 grams of S-1 with 0.852 grams of myrisitc acid and 30 grams of ethyl acetate. The solution was stirred on a hot plate for about 5 minutes.

The aqueous phase was prepared by combining 3.0 grams of a 10% solution of Alkanol XC with 17.6 grams of a 11.36% solution of Type IV gelatin in water. 26.7 grams of distilled water was then added and the solution was stirred on a hot plate for about five minutes.

The aqueous phase was combined with the oil phase and the mixture was stirred. The mixture was then passed three times through a colloid mill to obtain the dispersion. The ethyl acetate was removed by evaporation under reduced pressure. The dispersions were then coated in the format shown below (the numbers indicate the amount of each component in mg/m²).

| Scavenger Layer | | |
|---|---|---|
| 124 S-1 | 269 Gel | |

| Emulsion Layer | | |
|---|---|---|
| 353 M-20 | 807 Gel | 172 Ag |

| Scavenger Layer | | |
|---|---|---|
| 124 S-1 | 269 Gel | |

The coating format also contains a UV protection layer and an overcoat (not shown). Unexposed strips were processed in standard RA-4 developer and then stored at room temperature. The strips were analyzed for residual coupler as a function of time. A set of unprocessed strips were stored at room temperature for the same length of time to check for reaction between coupler and S-1 in raw stock. A second set of unexposed strips were processed in standard RA-4 developer and then subjected to two weeks of 50 klux high intensity sunshine radiation after storage at room temperature for two weeks. The change in Status A blue density was measured. The results are shown below.

| **M-20 remaining in mg/m**^{**2**} | | | | **Delta Blue Density** |
|---|---|---|---|---|
| Raw Stock | | Processed | | |
| after 2wk RT | after 4wk | after 2wk | after 4wk | |
| 325 | 310 | 322 | 315 | 0.13 |

### Example 5: (invention)

A dispersion of the magenta coupler M-20 was made in the same manner as described under Example 4.

A dispersion of the invention compound SI-20 was made in the following manner.

3.0 grams of SI-20 was combined with 0.2 grams of sodium dodecyl sulfate, 3.0 grams of poly(vinylpyrrolidone) and 54 grams of distilled water to constitute the slurry. The slurry was combined with 90 grams of zirconia beads and placed in a Sweco mill to grind for five days. The slurry was then separated from the zirconia beads by filtration. The final dispersion was prepared by combining 24 grams of a 11.36% solution of Type IV gelatin in water with 6.9 grams of distilled water and 44.1 grams of the ground slurry.

The dispersions were coated in the format shown below. As before, the numbers refer to coverages in mg/m²:

| Scavenger Layer | | | |
|---|---|---|---|
| 154 SI-20 | | 269 Gel | |

| Emulsion Layer | | | |
|---|---|---|---|
| 353 M-20 | 807 Gel | | 172 Ag |

| Scavenger Layer | | | |
|---|---|---|---|
| 154 SI-20 | | 269 Gel | |

This coating also contained a UV protection layer and overcoat (not shown). Strips based on the coating were subjected to the same set of tests as in Example 4. The results are summarized below:

| **M-20 remaining in mg/m**^{**2**} | | | | **Delta Blue Density** |
|---|---|---|---|---|
| Raw Stock | | Processed | | |
| after 2wk RT | after 4wk | after 2wk | after 4wk | |
| 308 | 306 | 160 | 150 | 0.05 |

A comparison of the numbers from Example 4 and Example 5 clearly indicates that the invention results in a substantial drop in the amount of coupler present in unexposed regions of a coating after processing and this translates to a very significant reduction in photochemical yellowing.

### Example 6:

A dispersion of the epoxide compound SI-20 was prepared in the following manner.

2.0 grams of SI-20 was combined with 2.0 grams of poly(vinylpyrrolidone) (Kollidon 25 grade from BASF), 0.11 grams of sodium dodecyl sulfate and 20.04 grams of distilled water to constitute the slurry. This was combined with 80.57 grams of zirconia beads and placed on a Sweco mill to grind for five days. The slurry was then separated from the beads by filtration. An aqueous solution of gelatin was prepared by combining 6.88 grams of a 24% solution of Type IV gelatin with 8.33 grams of distilled water. The pH of the gelatin solution was adjusted to 5.0. The latter was then combined with 25 grams of the ground slurry to constitute the dispersion.

A dispersion of the magenta coupler M-41 was prepared in the following manner. 14.73 grams of tri(2-ethylhexyl) phosphate was heated as coupler solvent to a temperature of 120°C and combined with 11.35 grams of image stabilizer STG-A1 and 3.4 grams of ST-1. This was then combined with 12.75 grams of coupler M-41 to constitute the oil phase. The aqueous phase was prepared by mixing 88.5 grams of a 24% w/w solution of Type IV gelatin with 21.2 grams of a 10% w/w solution of the surfactant Alkanol XC and 273 grams of distilled water. The latter was then heated to 70°C. The oil phase was then combined with the aqueous phase and the mixture passed twice through a microfluidizer at a pressure of 69 MPa (10,000 psi) at 70°C to obtain the dispersion.

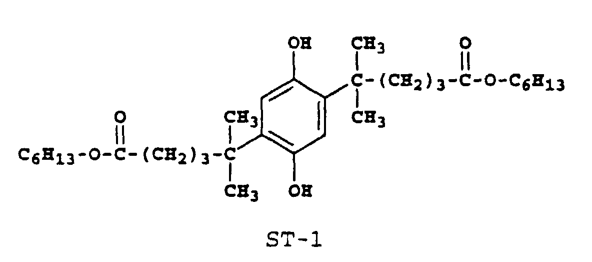

A dispersion of M-41 was also prepared with the image stabilizer ST-2 (control) in place of STG-A1.

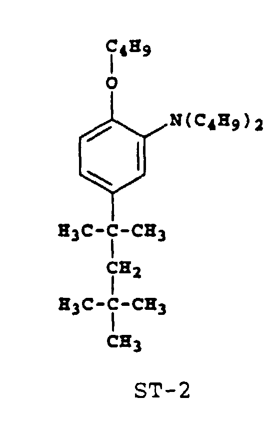

A given weight of dispersion of M-41 was then mixed with an equal weight of dispersion of epoxide SI-20 and the mixture was incubated in a water bath at 40°C. After 26 hours the mixture was analyzed by high perfomance liquid chromatography (HPLC) to determine the extent of reaction between SI-20 and M-41. The results are as follows:

| Image Stabilizer in M-41 disp. | %loss M-41 | %loss SI-20 |
|---|---|---|
| ST-2 (control) | 38.1 | 11.1 |
| STG-A1 (invention) | 89.5 | 31.4 |

It is clear that the extent of reaction in a given time between the magenta coupler M-41 and the terminal epoxide compound SI-20 is markedly greater using the formulation comprising an image stabilizer of formula STG-A compared to the control.

### Example 7:

A dispersion containing the magenta coupler M-24 was prepared in the same manner as the dispersion for M-41 described in Example 6 except that a mixture of 8.53 grams of STG-A1 and 2.83 grams of ST-3 was used in place of the 11.35 grams of STG-A1.

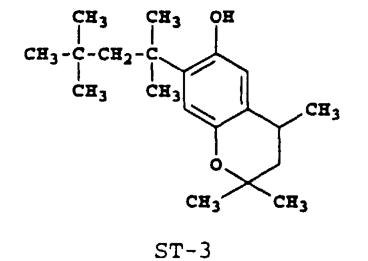

The dispersion of M-24 and the dispersion of SI-20 described previously (Example 6) were mixed with emulsion and coated on a paper supports using the layer formats shown below.

| | | | | | | |
|---|---|---|---|---|---|---|
| 630 Gel | | | 215 SI-20 | | 630 Gel | |
| 172 Ag | 430 M-24 | 1270 Gel | 172 Ag | 430 M-24 | | 1270 Gel |
| | 753 Gel | | 215 SI-20 | | 753 Gel | |
| A | | | B | | | |

All numbers refer to coverages in mg/m². The coatings also contain a UV protection layer and an overcoat (not shown). 35mm strips from these coatings were exposed using a 21 step tablet and processed using the standard RA-4 process. The processed strips were then stored at room temperature for six weeks and then subjected to 3 weeks of 50 Klux high intensity daylight radiation. The change in blue reflection density in the Dmin (unexposed) region and the change in green density from 1.0 following irradiation were noted. The results are as follows:

| | Δ Blue Dmin | Δ Green 1.0 |
|---|---|---|
| Coating A (control) | 0.19 | 0.21 |
| Coating B (invention) | 0.10 | 0.29 |

It is clear that a combination of epoxide compound and an image stabilizer of the type STG-A gives a much superior position with respect to photochemical yellowing while maintaininig a good position with repect to dye fade.

### Example 8:

Additional dispersions of M-41 were prepared according to the method of Example 6 but using image stabilizers ST-4 and ST-5 as controls in place of STG-A1.

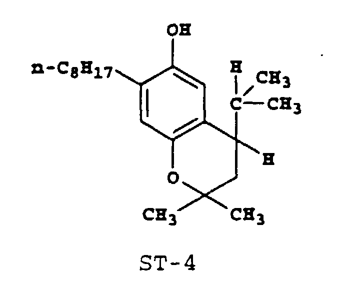

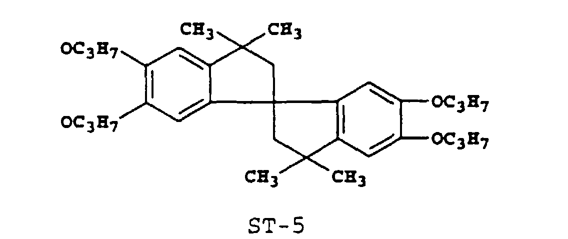

As before, a given weight of dispersion of M-41 was mixed with an equal weight of dispersion of epoxide SI-20 and the mixture was then incubated in a water bath at 40°C. After 26 hours the mixture was analyzed by high performance liquid chromatography (HPLC) to determine the extent of reaction between SI-20 and M-41. The results are as follows:

| Image Stabilizer in M-41 dispersion | % loss M-41 |
|---|---|
| ST-4 (control) | 59.0 |
| ST-5 (control) | 34.0 |

It is clear that in both cases the extent of reaction in a given time between the magenta coupler and the terminal epoxide compound SI-20 is significantly less than that seen using the formulation of our invention.

### Example 9:

A dispersion of the terminal epoxy compound SI-20 (dispersion A) was prepared in the following manner.

2.0 grams of SI-20 was combined with 2.0 grams of polyvinylpyrrolidone (PVP K-25 from BASF), 0.11 grams of sodium dodecyl sulfate, 29.04 grams of water and 80.57 grams of zirconia beads to constitute the slurry. The slurry was placed on a sweco mill for five days. The zirconia beads were then removed from the slurry by filtration. The aqueous phase was prepared by combining 6.88 grams of a 24% w/w solution of Type IV gelatin with 8.33 grams of distilled water. The pH of the aqueous phase was adjusted to 5.0. 25.0 grams of the ground slurry was then mixed with said aqueous phase to constitute the dispersion.

A second dispersion of the terminal epoxy compound SI-20 (dispersion B) was prepared in the same manner as described above except that 0.5 grams of the high logP liquid tri(2-ethylhexyl) phosphate (logP = 9.49) was added to the slurry prior to milling.

A dispersion of the magenta coupler M-24 was prepared in the following manner. 14.73 grams of tri(2-ethylhexyl) phosphate was heated as coupler solvent to a temperature of 120°C and combined with a mixture of 8.53 grams of image stabilizer STG-A1 and 2.83 grams of ST-3 and 3.4 grams of ST-1. This was then combined with 12.75 grams of coupler M-24 to constitute the oil phase. The aqueous phase was prepared by mixing 88.5 grams of a 24% w/w solution of Type IV gelatin with 21.2 grams of a 10% w/w solution of the surfactant Alkanol XC and 273 grams of distilled water. The latter was then heated to 70°C. The oil phase was then combined with the aqueous phase and the mixture passed twice through a microfluidizer at a pressure of 69 MPa (10,000 psi) at 70°C to obtain the dispersion.

The dispersions were then coated on a support in the layer format shown below.

| | | | |
|---|---|---|---|
| 215 SI-20 | | 630 Gel | |
| 172 Ag | 430 M24 | | 1270 Gel |
| 215 SI-20 | | 753 Gel | |

All numbers refer to coverages in mg/m². The coatings also contain a UV protection layer and an overcoat(not shown). A set of 35mm strips from these coatings were exposed using a 21 step tablet and processed using the standard RA-4 process. A second set of 35mm strips were treated in the same manner except that these strips were first incubated in an oven at 49°C and 50% RH for one week prior to exposure and processing. The difference in the green reflection density between the two sets of strips at an exposure corresponding to a density of 1.0 in the first set of strips was noted.

| | Δ Green 1.0 |
|---|---|
| Dispersion A | 0.19 |
| Dispersion B | 0.08 |

It is clear that the method of the invention using a high logP component results in coatings containing solid particle dispersions that are more stable during raw stock keeping at elevated temperatures.

### Example 10:

A dispersion of a solid terminal epoxide, SI-20 (logP = 4.5, melting point 77°C) was prepared in the following manner.

3.0 grams of SI-20 was combined with 1.0 gram of the water-immiscible organic solvent tri(2-ethylhexyl) phosphate. The mixture was heated until the oil phase was homogeneous. 26.0 grams of a 12% w/w solution of Type IV gelatin in water was combined with 43.8 grams of water and 4.0 grams of Alkanol XC to constitute the aqueous phase. The aqueous phase was maintained at 70°C. The oil phase was combined with the aqueous phase while stirring. The mixture was then passed twice through the microfluidizer at a pressure of 69 MPa (10,000 psi) to obtain the dispersion.

A dispersion of the low-melting point epoxide compound SI-25 (logP = 4.3, melting point less than -160°C and a liquid at room temperature, (25°C)) was prepared in the same manner as above.

Both dispersions were placed in a constant temperature bath set at 45°C. It was found that the dispersion of the solid compound SI-20 disintegrated (with significant settling) after just 6 hours. However, the dispersion of the liquid compound SI-25 showed no evidence of settling or macroscopic coagulation even after 12 hours.

### Example 11

A dispersion of the magenta coupler M-24 was prepared in the following manner. 14.73 grams of the coupler solvent tri(2-ethylhexyl) phosphate was heated to a temperature of 120°C and combined with 8.50 grams of image stabilizer STG-A1, 2.83 grams of ST-3 and 3.4 grams of ST-1. This was then combined with 12.75 grams of M-24 to constitute the oil phase. The aqueous phase was prepared by mixing 88.5 grams of a 24% w/w solution of Type IV gelatin with 21.2 grams of a 10% w/w solution of the surfactant Alkanol XC and 273 grams of distilled water. The latter was then heated to 70°C. The oil phase was then combined with the aqueous phase and the mixture passed twice through a microfluidizer at a pressure of 69 MPa (10,000 psi) at 70°C to obtain the dispersion.

A dispersion of the epoxide compound SI-25 was made in the same manner as described in Example 6 except that 2.0 grams of tri(2-ethylhexyl) phosphate was used instead of 1.0 gram.

The dispersion of M-24 and the dispersion of SI-25 were then coated on a paper support using the layer format shown below.

| | | |
|---|---|---|
| 215 SI-25 | | 630 Gel |
| 172 AG | 430 M-24 | 1270 Gel |
| 215 SI-25 | | 753 Gel |

All numbers refer to coverages in mg/m². The coatings also contain a UV protection layer and an overcoat ( not shown ). A second set of coatings containing no SI-25 were prepared to serve as a control. 35mm strips from these coatings were exposed using a 21 step tablet and processed using the standard RA-4 process. The processed strips were then stored at room temperature for six weeks and then subjected to 2 weeks of 50 Klux high intensity daylight radiation. The change in blue reflection density in the Dmin (unexposed) region was noted. The results are as follows:

| | **D Blue Dmin** |
|---|---|
| **Control** | **0.22** |
| **Invention** | **0.10** |

It is clear that the method of the invention employing low melting point epoxy compounds results in significant improvement in photochemical yellowing.

## Claims

1. A compound having the structural formula: 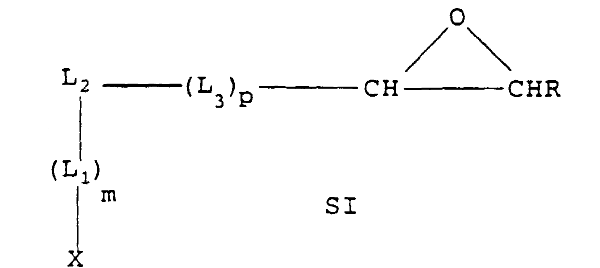 wherein:
R is H, an alkyl group, or an aryl group;
L₁ is an alkyl group or an aryl group;
L₂ is -O-, -CO-, -S-, -SO₂-, -PO₂-, -CO₂-, -NHCO- or -NHSO₂-, wherein L₂ may be orientated in either direction;
L₃ is an alkyl group;
m is 0 or 1;
p is 0 or 1;
and X is 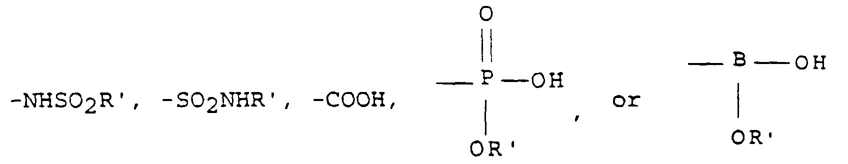
wherein R' is H or an alkyl or aryl group, with the proviso that where L₂ comprises an ionizable group, X may also be an alkyl group or an aryl group.

2. A compound according to claim 1, wherein m and p are each 1, L₁ is phenyl, L₂ is -O-, -CO-, -SO₂-, -PO₂-, or -CO₂-, L₃ is a linear or branched alkyl group, X is 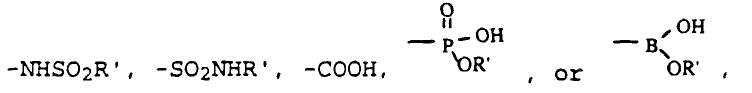 and R' is an alkyl or aryl group.

3. A compound according to cliam 1, wherein X is 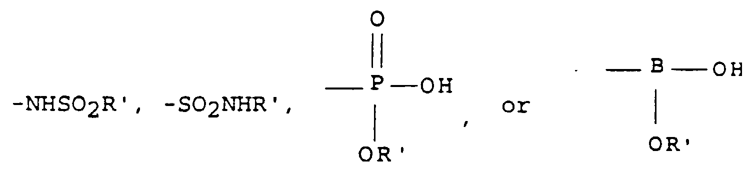 wherein R' is H or an alkyl or aryl group, with the proviso that where L₂ comprises an ionizable group, X may also be an alkyl group or an aryl group; and wherein the compound has a melting point of less than about 50°C.

4. A compound according to claim 3, having the generic structural formula SI-B: 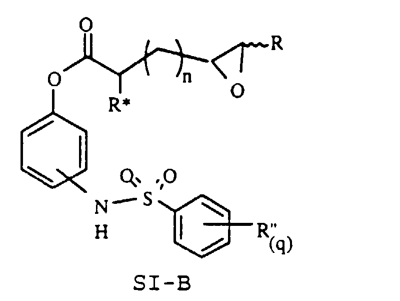 wherein: R* is H or an alkyl or aryl group; n is from 1 to about 20; q is 1, 2, or 3; and each R'' is H, an alkoxide group, a phosphate group, a sulfate group, a sulfonamide group, a sulfone group, a halogen atom, or an alkyl group; with the proviso the appended sulfonamido group is in a meta or para position with respect to the -CO₂- linking group, and with the further proviso when the appended sulfonamido group is in the para position each R'' is H, or at least one appended R'' group which is not H is in the meta or ortho position with respect to the -NHSO₂- linking group, or R* is not H.

5. A compound according to claim 1, wherein the compound has the general structural formula: 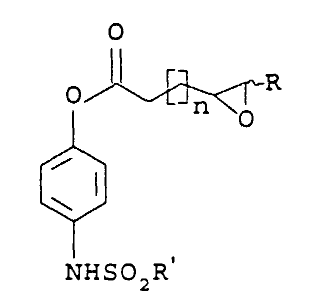 wherein:
R is H or an alkyl or aryl group;
R' is H or an alkyl or aryl group;
and n is 2 to 20.

6. A compound according to claim 1, wherein the compound has the formula: 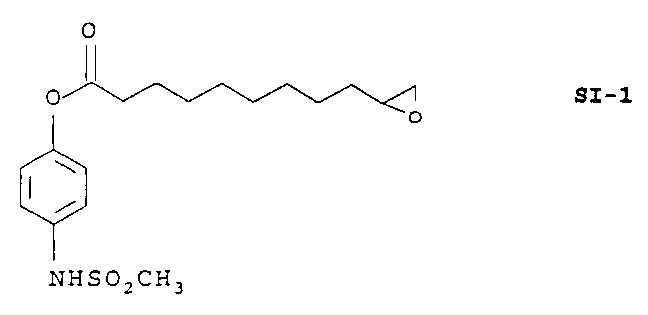 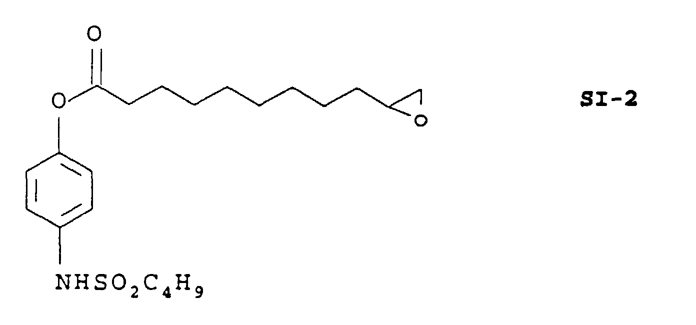 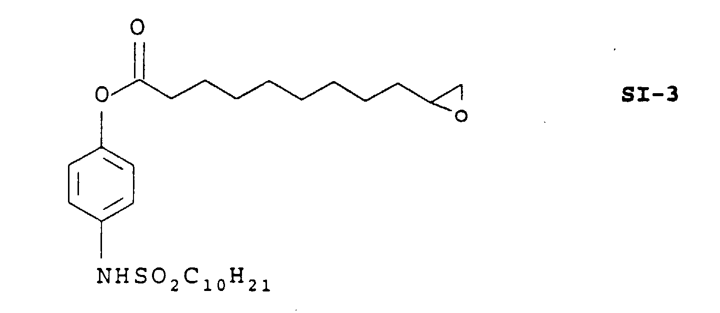 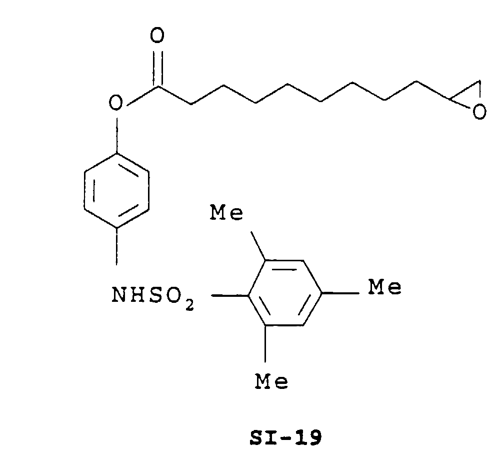 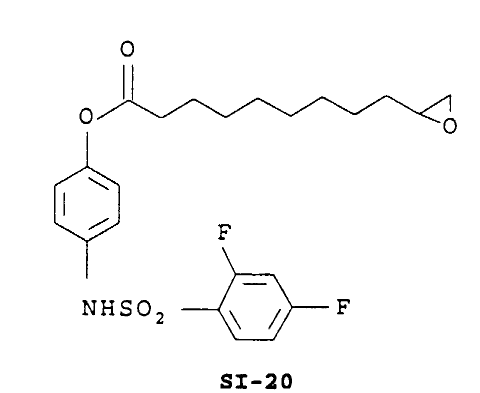 **OR**

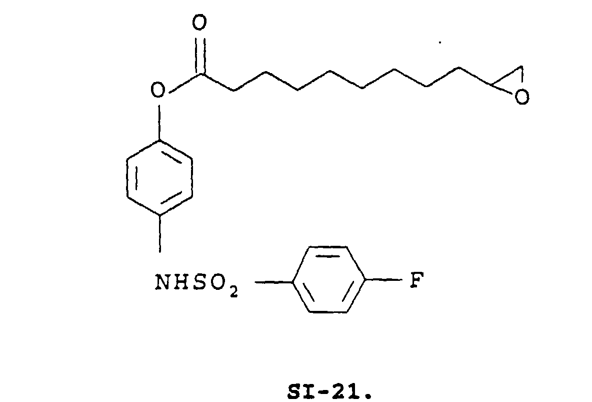

7. A photographic element comprising a support having coated thereon:
(a) a photosensitive first layer comprising
(i) a silver halide emulsion and
(ii) a magenta coupler dispersed therein; and
(b) a second layer comprising an epoxy compound according to any one of claims 1 through 6.

8. A photographic element according to claim 7, wherein the photosensitive first layer further comprises an image stabilizer of the following formula STG-A: 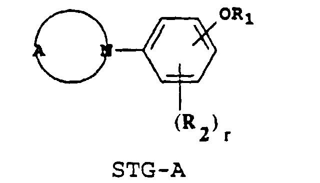 wherein R₁ represents an alkyl group, a cycloalkyl group, an alkenyl phenyl group, an aryl group, a heterocyclic group, an acyl group, a bridged hydrocarbon group, an alkyl sulfonyl group or an aryl sulfonyl group; R₂ represents a group capable of being substituted on the benzene ring; r represents an integer between 0 and 4; and A represents a group of non metal atoms necessary for the formation of a 5 to 8 membered ring together with the nitrogen atom.

9. A photographic element according to claim 8, wherein A comprises an electron withdrawing group.

10. A photographic element according to claim 8, wherein A comprises a sulfoxide group.

11. A process for preparing a solid particle dispersion of an epoxy compound of formula SI according to any one of claims 1, 2, 5, or 6, comprising the steps of:
(a) forming a coarse aqueous slurry of solid particles of said compound; and
(b) milling said slurry in the presence of a hydrophobic, photographically inert, liquid second component which has a logP_{(calc)} greater than about 6.0 for time sufficient to provide particles of the desired average particle size.

12. A process according to claim 11, wherein the amount of the second component used in step (b) is less than or equal to one fourth of the weight of the compound of formula SI.

13. A process of forming an aqueous dispersion of an epoxy compound according to claim 3 or 4, comprising adding the compound in a liquid state to an aqueous solution, and directly dispersing the compound in the aqueous solution.

## Patentansprüche

1. Verbindung, **gekennzeichnet durch** die Strukturformel: 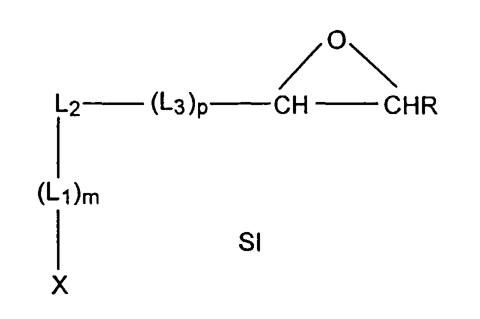 wobei
R ein H-Atom, eine Alkyl- oder Arylgruppe ist;
L₁ eine Alkyl- oder Arylgruppe ist;
L₂ -O-, -CO-, -S-, -SO₂ -, -PO₂ -CO₂ -, -NHCO- oder -NHSO₂- ist, wobei L₂ in beiden Richtungen orientiert sein kann;
L₃ eine Alkylgruppe ist;
m 0 oder 1 ist;
p 0 oder 1 ist;
und X ist 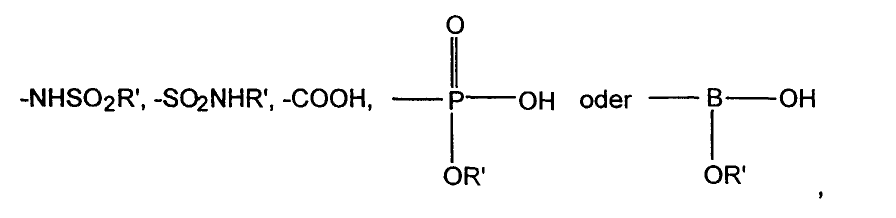 wobei R' ein H-Atom, eine Alkyl- oder Arylgruppe ist, mit dem Vorbehalt, dass dort, wo L₂ eine ionisierbare Gruppe ist, X auch eine Alkyl- oder Arylgruppe sein kann.

2. Verbindung nach Anspruch 1, dadurch gekennzeichnet, dass m und p jeweils 1 sind, L₁ eine Phenylgruppe ist, L₂ -O-, -CO-, -SO₂-, -PO₂- oder -CO₂- ist, L₃ eine lineare oder verzweigte Alkylgruppe darstellt, X 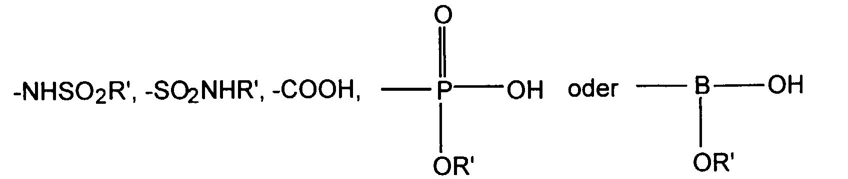 ist und R' eine Alkyl- oder Arylgruppe ist.

3. Verbindung nach Anspruch 1, dadurch gekennzeichnet, dass X 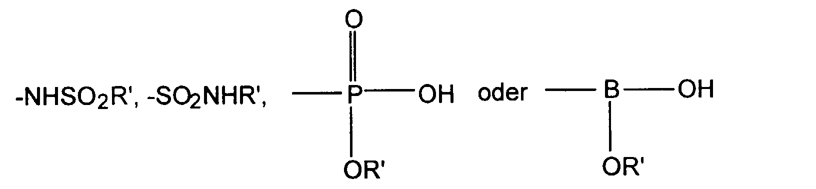 ist, wobei R' ein H-Atom, eine Alkyl- oder Arylgruppe ist, mit dem Vorbehalt, dass dort, wo L₂ eine ionisierbare Gruppe ist, X auch eine Alkyl- oder Arylgruppe sein kann; und wobei die Verbindung einen Schmelzpunkt unterhalb von etwa 50 °C aufweist.

4. Verbindung nach Anspruch 3, gekennzeichnet durch die allgemeine Strukturformel SI-B: 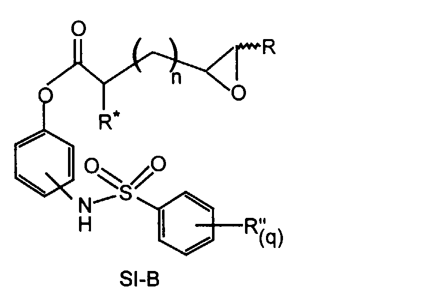 wobei R* ein H-Atom oder eine Alkyl- oder Arylgruppe ist; n zwischen 1 und 20 liegen kann; q 1, 2 oder 3 ist und R" jeweils ein H-Atom, eine Alkoxidgruppe, Phosphatgruppe, Sulfatgruppe, Sulfonamidgruppe, Sulfongruppe, Halogen oder eine Alkylgruppe ist; mit dem Vorbehalt, dass sich die anhängende Sulfonamidgruppe in meta- oder para-Position zur -CO₂-Brückengruppe befindet, und mit dem weiteren Vorbehalt, dass, wenn die anhängende Sulfonamidgruppe die para-Position einnimmt, jede R"-Gruppe ein H-Atom ist, oder mindestens eine anhängende R"-Gruppe, die nicht H ist, befindet sich bezüglich der -NHSO₂-Brückengruppe in meta- oder ortho-Position, oder R* ist nicht H.

5. Verbindung nach Anspruch 1, gekennzeichnet durch die allgemeine Strukturformel: 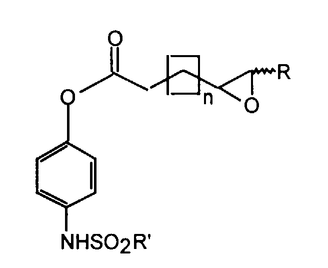 wobei
R ein H-Atom oder eine Alkyl- oder Arylgruppe ist;
R' ein H-Atom oder eine Alkyl- oder Arylgruppe ist;
und n zwischen 2 und 20 liegt.

6. Verbindung nach Anspruch 1, gekennzeichnet durch die Formeln: 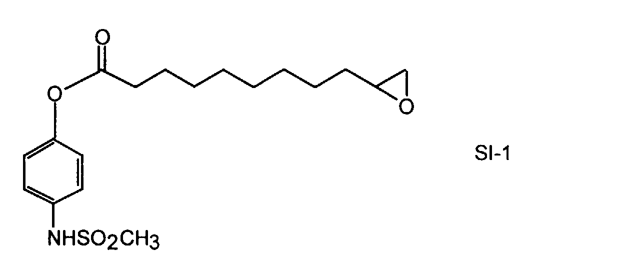 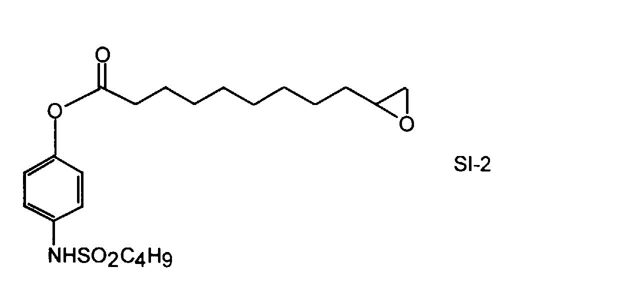 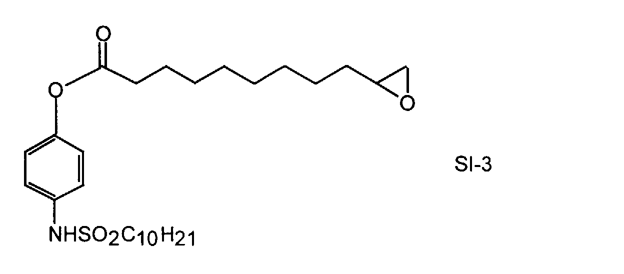 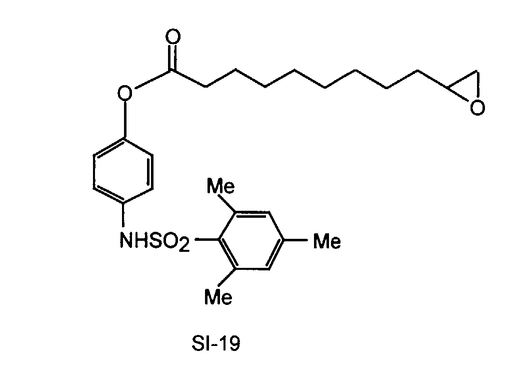 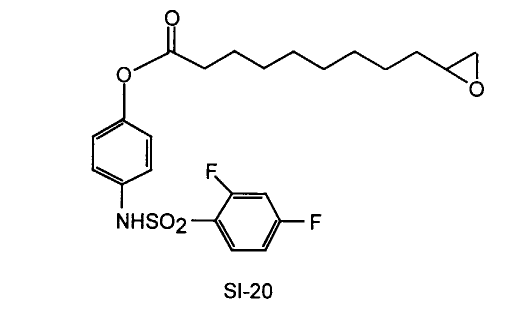 oder

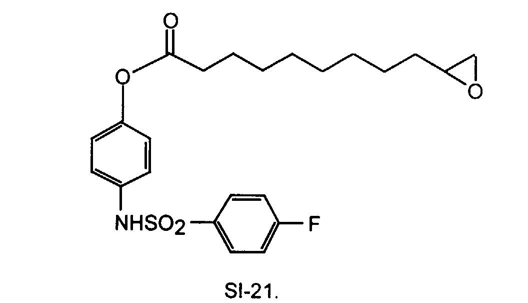

7. Fotografisches Element, **gekennzeichnet durch** einen Träger, der
(a) mit einer lichtempfindlichen ersten Schicht beschichtet ist, die
(i) eine Silberhalogenidemulsion und
(ii) in dieser dispergiert einen Purpurkuppler umfasst, und
(b) mit einer zweiten Schicht beschichtet ist, die eine Epoxy-Verbindung nach Anspruch 1 bis 6 enthält.

8. Fotografisches Element nach Anspruch 7, dadurch gekennzeichnet, dass die lichtempfindliche erste Schicht zusätzlich einen Bildstabilisator der folgenden Formel STG-A umfasst: 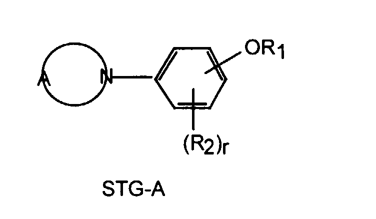 wobei R₁ eine Alkylgruppe, Cycloalkylgruppe, Alkenylphenylgruppe, Arylgruppe, heterocyclische Gruppe, Acylgruppe, überbrückte Kohlenwasserstoffgruppe, Alkylsulfonylgruppe oder Arylsulfonylgruppe ist; R₂ eine Gruppe ist, die als Substituent am Benzolring fungieren kann; r eine ganze Zahl zwischen 0 und 4 ist und A eine Gruppe von Nichtmetallatomen darstellt, die für die Bildung eines 5-bis 8-gliedrigen Rings, unter Einschluss des Stickstoffatoms, erforderlich ist.

9. Fotografisches Element nach Anspruch 8, dadurch gekennzeichnet, dass A eine elektronenziehende Gruppe umfasst.

10. Fotografisches Element nach Anspruch 8, dadurch gekennzeichnet, dass A eine Sulfoxidgruppe umfasst.

11. Verfahren zur Herstellung einer Feststoffteilchendispersion einer Epoxy-Verbindung der Formel Sl nach Anspruch 1, 2, 5 oder 6, **gekennzeichnet durch** folgende Schritte:
(a) Bildung einer grobkörnigen wässrigen Aufschlämmung von Feststoffteilchen der Verbindung und
(b) Vermahlung der Aufschlämmung in Gegenwart einer hydrophoben, fotografisch inerten, flüssigen zweiten Komponente mit einem logP_{(berechnet)}-Wert grösser als etwa 6,0, für eine Zeitdauer, die für die Bildung von Teilchen der gewünschten mittleren Teilchengrösse ausreicht.

12. Verfahren nach Anspruch 11, dadurch gekennzeichnet, dass die Menge der in Schritt (b) eingesetzten zweiten Komponente kleiner oder gleich einem Viertel des Gewichts der Verbindung der Formel ST ist.

13. Verfahren zur Bildung einer wässrigen Dispersion einer Epoxy-Verbindung nach Anspruch 3 oder 4, dadurch gekennzeichnet, dass die Verbindung in flüssigem Zustand der wässrigen Lösung zugesetzt wird und die Verbindung unmittelbar in der wässrigen Lösung dispergiert wird.

## Revendications

1. Composé ayant la formule développée : 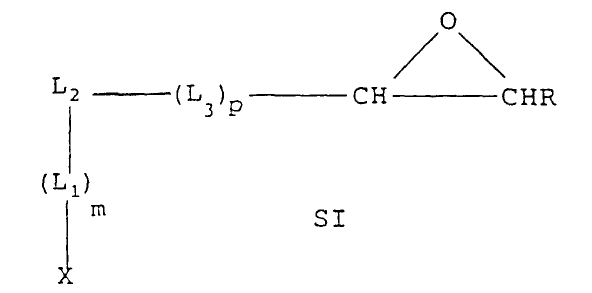 dans laquelle :
R représente H, un groupe alkyle ou un groupe aryle ;
L₁ représente un groupe alkyle ou un groupe aryle ;
L₂ représente -O-, -CO-, -S-, -SO₂-, -PO₂-, -CO₂-, -NHCO- ou -NHSO₂-, où L₂ peut être orienté dans l'une ou l'autre direction ;
L₃ représente un groupe alkyle ;
m est égal à 0 ou 1 ;
p est égal à 0 ou 1 ;
et X représente 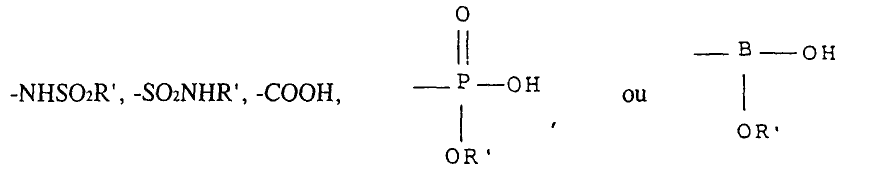
dans lesquels R' représente H, un groupe alkyle ou un groupe aryle, à condition que, lorsque L₂ comprend un groupe ionisable, X puisse également représenter un groupe alkyle ou un groupe aryle.

2. Composé selon la revendication 1, dans lequel m et p sont chacun égaux à 1, L₁ représente un groupe phényle, L₂ représente -O-, -CO-, -SO₂-, -PO₂- ou -CO₂-, L₃ représente un groupe alkyle linéaire ou ramifié, X représente -NHSO₂R', -SO₂NHR', 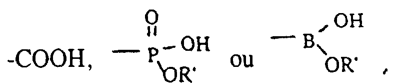 , et R' représente un groupe alkyle ou aryle.

3. Composé selon la revendication 1, dans lequel X représente -NHSO₂R', 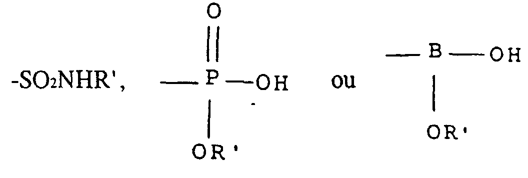 dans lesquels R' représente H ou un groupe alkyle ou aryle, à condition que, lorsque L₂ comprend un groupe ionisable, X puisse également représenter un groupe alkyle ou un groupe aryle ; et dans lequel le composé a un point de fusion inférieur à 50 °C environ.

4. Composé selon la revendication 3, ayant la formule développée générique SI-B : 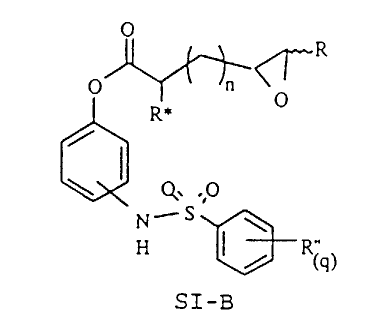 dans laquelle : R* représente H ou un groupe alkyle ou aryle ; n est compris entre 1 et 20 environ ; q est égal à 1, 2 ou 3 ; et chaque R" représente H, un groupe alcoxyde, un groupe phosphate, un groupe sulfate, un groupe sulfonamide, un groupe sulfone, un atome d'halogène ou un groupe alkyle ; à condition que le groupe sulfonamido rattaché soit en position méta ou para par rapport au groupe de liaison -CO₂-, et à condition aussi que, lorsque le groupe sulfonamido rattaché est en position para, chaque R" représente H, ou au moins l'un des groupes R" rattachés qui ne représente pas H est en position méta ou ortho par rapport au groupe de liaison -NHSO₂-, ou R* ne représente pas H.

5. Composé selon la revendication 1, dans lequel le composé a la formule développée générale : 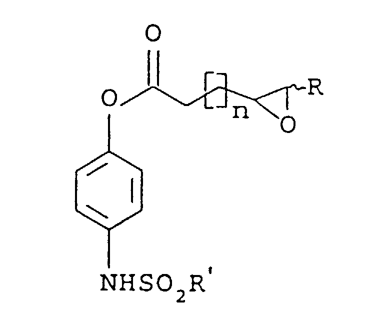 dans laquelle :
R représente H ou un groupe alkyle ou aryle ;
R' représente H ou un groupe alkyle ou aryle ;
et n est compris entre 2 et 20.

6. Composé selon la revendication 1, dans lequel le composé a la formule : 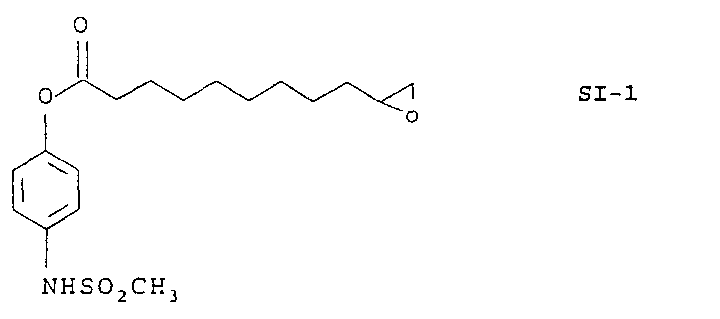 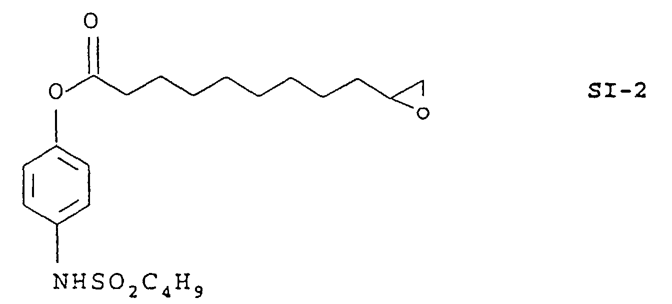 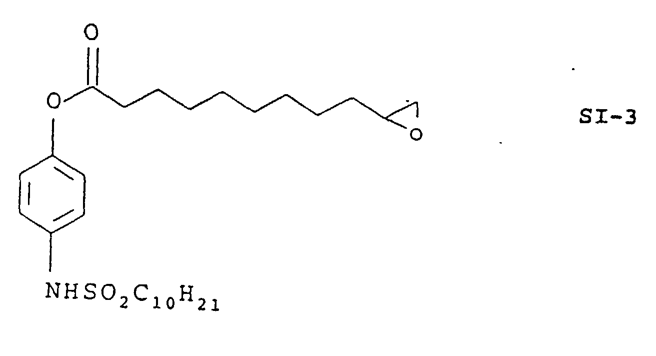 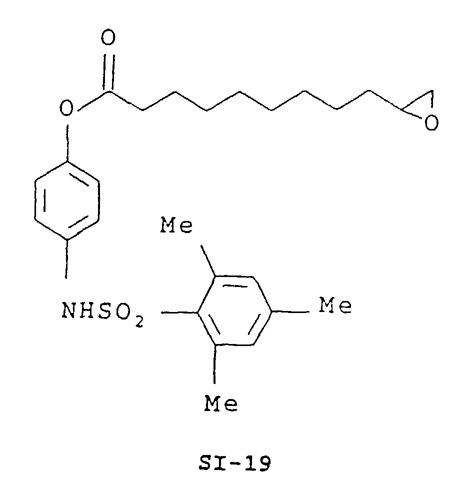 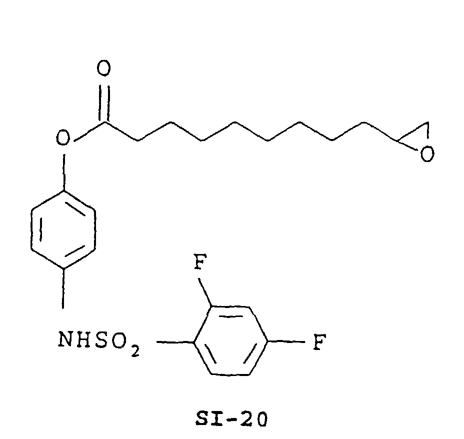 OR

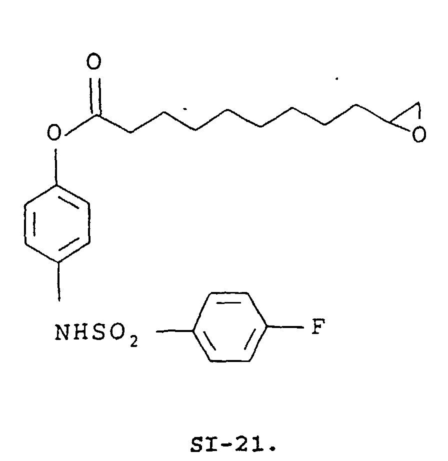

7. Elément photographique comprenant un support sur lequel sont appliquées :
(a) une première couche photosensible comprenant
(i) une émulsion aux halogénures d'argent et
(ii) un coupleur magenta dispersé dans cette émulsion ; et
(b) une seconde couche comprenant un composé époxy selon l'une quelconque des revendications 1 à 6.

8. Elément photographique selon la revendication 7, dans lequel la première couche photosensible comprend aussi un stabilisateur d'image ayant la formule STG-A suivante : 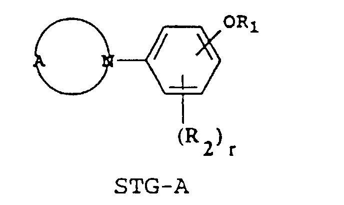 dans laquelle R₁ représente un groupe alkyle, un groupe cycloalkyle, un groupe alcénylphényle, un groupe aryle, un hétérocycle, un groupe acyle, un groupe hydrocarboné ponté, un groupe alkylsulfonyle ou un groupe arylsulfonyle ; R₂ représente un groupe pouvant être substitué sur le cycle aromatique ; r représente un nombre entier entre 0 et 4 ; et A représente un groupe d'atomes non métalliques nécessaires pour former, avec l'atome d'azote, un noyau à 5 à 8 membres .

9. Elément photographique selon la revendication 8, dans lequel A comprend un groupe attracteur d'électrons.

10. Elément photographique selon la revendication 8, dans lequel A comprend un groupe sulfoxyde.

11. Procédé de préparation d'une dispersion de particules solides d'un composé époxy de formule SI selon l'une quelconque des revendications 1, 2, 5 ou 6, comprenant les étapes de :
(a) formation d'une bouillie aqueuse de particules solides grossière dudit composé ; et
(b) broyage de ladite bouillie en présence d'un second composant liquide hydrophobe, photographiquement inerte, qui a un logP_{(calc)} supérieur à 6,0 environ, pendant une durée suffisante pour obtenir des particules ayant la granulométrie moyenne souhaitée.

12. Procédé selon la revendication 11, dans lequel la quantité du second composant utilisé dans l'étape (b) est inférieure ou égale au quart du poids du composé de formule SI.

13. Procédé de préparation d'une dispersion aqueuse d'un composé époxy selon la revendication 3 ou 4, comprenant l'addition du composé à l'état liquide à une solution aqueuse, et la dispersion directe du composé dans la solution aqueuse.
